(19) Europäisches Patentamt
European Patent Office
Office européen des brevets

(11) EP 1 228 059 B1

(12) EUROPÄISCHE PATENTSCHRIFT

(45) Veröffentlichungstag und Bekanntmachung des Hinweises auf die Patenterteilung:
**03.08.2005 Patentblatt 2005/31**

(21) Anmeldenummer: **00969529.7**

(22) Anmeldetag: **21.10.2000**

(51) Int Cl.[7]: **C07D 401/04**, C07D 471/04, A61K 31/445, A61P 29/00

(86) Internationale Anmeldenummer:
**PCT/EP2000/010391**

(87) Internationale Veröffentlichungsnummer:
**WO 2001/032648 (10.05.2001 Gazette 2001/19)**

(54) **NEUE CYCLOPROPANE ALS CGRP-ANTAGONISTEN, DIESE VERBINDUNGEN ENTHALTENDE ARZNEIMITTEL UND VERFAHREN ZU IHRER HERSTELLUNG**

NOVEL CYCLOPROPANES AS CGRP ANTAGONISTS, MEDICAMENTS CONTAINING SAID COMPOUNDS AND METHOD FOR THE PRODUCTION THEREOF

NOUVEAUX CYCLOPROPANES UTILISES COMME ANTAGONISTES DE CGRP, MEDICAMENTS CONTENANT LESDITS COMPOSES ET PROCEDES PERMETTANT DE LES PREPARER

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **29.10.1999 DE 19952147**

(43) Veröffentlichungstag der Anmeldung:
**07.08.2002 Patentblatt 2002/32**

(73) Patentinhaber: **Boehringer Ingelheim Pharma GmbH & Co.KG**
**55218 Ingelheim am Rhein (DE)**

(72) Erfinder:
- **EBERLEIN, Wolfgang**
  **88400 Biberach (DE)**
- **ENGEL, Wolfhard**
  **88400 Biberach (DE)**
- **RUDOLF, Klaus**
  **88447 Warthausen (DE)**
- **DOODS, Henri**
  **88447 Warthausen (DE)**
- **HALLERMAYER, Gerhard**
  **88437 Maselheim-Sulmingen (DE)**
- **BAUER, Eckhart**
  **88400 Biberach (DE)**

(56) Entgegenhaltungen:
**WO-A-98/09630** **WO-A-99/47517**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 228 059 B1

## Beschreibung

Gegenstand der vorliegenden Erfindung sind neue Cyclopropane der allgemeinen Formel

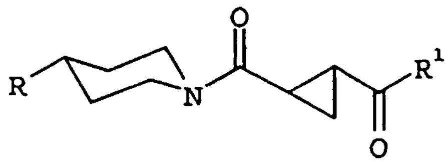

(I),

deren Tautomere, deren Diastereomere, deren Enantiomere, deren Gemische und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In der obigen allgemeinen Formel (I) bedeuten

R einen gesättigten, einfach oder zweifach ungesättigten 5- bis 7-gliedrigen Aza-, Diaza-, Triaza-, Oxaza-, Thiaza-, Thiadiaza- oder S,S-Dioxido-thiadiaza-Heterocyclus,

wobei die vorstehend erwähnten Heterocyclen über ein Kohlenstoff- oder Stickstoffatom verknüpft sein und ein oder zwei Carbonylgruppen benachbart zu einem Stickstoffatom enthalten können,

an einem der Stickstoffatome durch eine Alkylgruppe substituiert sein können,

an einem oder an zwei Kohlenstoffatomen durch eine verzweigte oder unverzweigte Alkylgruppe, durch eine Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolyl-Gruppe substituiert sein können, wobei die Substituenten gleich oder verschieden sein können,

und wobei eine olefinische Doppelbindung eines der vorstehend erwähnten ungesättigten Heterocyclen mit einem Benzol-, Pyridin-, Diazin-, 1,3-Oxazol-, Thiophen-, Furan-, Thiazol-, Pyrrol-, N-Methyl-pyrrol-, Chinolin-, Imidazol- oder N-Methyl-imidazol-Ring kondensiert sein kann,

wobei die in R enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolyl-Gruppen sowie benzo-, thieno-, pyrido- und diazinokondensierten Heterocyclen im Kohlenstoffgerüst zusätzlich durch Fluor-, Chlor- oder Bromatome, durch Alkyl-, Dialkylaminoalkoxy-, Nitro-, Alkylthio-, Alkylsulfinyl-, Alkylsulfonyl-, Alkylsulfonylamino-, Phenyl-, Trifluormethyl-, Alkoxycarbonyl-, Alkoxycarbonylalkyl-, Alkoxycarbonylalkoxy-, Hydroxycarbonylalkoxy-, Carboxy-, Carboxyalkyl-, Dialkylaminoalkyl-, Hydroxy-, Amino-, Acetylamino-, Propionylamino-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, (4-Morpholinyl)carbonyl-, (1-Pyrrolidinyl)carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Aminocarbonylaminoalkyl-, Alkylaminocarbonylamino-, Alkanoyl-, Cyan-, Trifluormethoxy-, Trifluormethylthio-, Trifluormethylsulfinyl-, Trifluormethylsulfonyl- oder Cycloalkylgruppen mit 3 bis 8 Kohlenstoffatomen,

durch 4- bis 8-gliedrige Alkyleniminogruppen, in denen eine Methylengruppe in 3-, 4- oder 5-Position durch ein Sauerstoffatom oder eine Methyliminogruppe ersetzt sein kann,

durch Alkoxygruppen, die in ω-Stellung durch einen 5- bis 7-gliedrigen Heteroalicyclus substituiert sein können, wobei der Heteroalicyclus über ein Kohlenstoff- oder Stickstoffatom verknüpft ist und ein oder zwei nicht direkt aneinander gebundene Heteroatome ausgewählt aus Sauerstoff und Stickstoff enthält,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenyl-, 1-Naphthyl-, 2-Naphthyl-, 1,2,3,4-Tetrahydro-1-naphthyl-, 1H-Indol-3-yl-, 1-Methyl-1H-indol-3-yl-, 1-Formyl-1H-indol-3-yl-, 4-Imidazolyl-, 1-Methyl-4-imidazolyl-, 2-Thienyl-, 3-Thienyl-, Thiazolyl-, 1H-Indazol-3-yl-, 1-Methyl-1H-indazol-3-yl-, Benzo[b]fur-3-yl-, Benzo[b]thien-3-yl-, Pyridinyl-, Chinolinyl- oder Isochinolinylgruppe,

wobei die vorstehend erwähnten aromatischen und heteroaromatischen Reste im Kohlenstoffgerüst zusätzlich durch Fluor-, Chlor- oder Bromatome, durch verzweigte oder unverzweigte Alkylgruppen, durch Cycloalkylgruppen mit 3 bis 8 Kohlenstoffatomen, durch Phenylalkyl-, Alkenyl-, Alkoxy-, Phenyl-, Phenylalkoxy-, Trifluormethyl-, Alkoxycarbonylalkyl-, Carboxyalkyl-, Alkoxycarbonyl-, Carboxy-, Dialkylaminoalkyl-, Dialkylaminoalkoxy-, Nitro-, Hydroxy-, Amino-, Acetylamino-, Propionylamino-, Methylsulfonyloxy-, Aminocarbonyl-, Alkylaminocarbonyl-, Dialkylaminocarbonyl-, Alkanoyl-, Cyan-, Tetrazolyl-, Phenyl-, Pyridinyl-, Thiazolyl-, Furyl-, Trifluormethoxy-, Trifluormethylthio-, Trifluormethylsulfinyl- oder Trifluormethylsulfonylgruppen mono-, di- oder trisubstituiert sein können und die Substituenten gleich oder verschieden sein können,

wobei die in den vorstehend genannten Gruppen enthaltenen Hydroxy-, Amino-, Indolyl- und Imidazolylgruppen mit den aus der Peptidchemie geläufigen Schutzresten substituiert sein können, vorzugsweise mit der Acetyl-, Benzyloxycarbonyl- oder tert.Butyloxycarbonyl-Gruppe, und

alle vorstehend genannten Alkyl- und Alkoxygruppen sowie die innerhalb der anderen genannten Reste vorhandenen Alkyl- oder Alkylenteile, sofern nichts anderes angegeben ist, 1 bis 5 Kohlenstoffatome umfassen können.

Unter den in den vorstehenden Definitionen genannten Schutzresten sind die aus der Peptidchemie geläufigen Schutzgruppen zu verstehen, nämlich

eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,

beispielsweise die Benzyloxycarbonyl-, 2-Nitro-benzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe,

eine Alkoxycarbonylgruppe mit insgesamt 1 bis 5 Kohlenstoffatomen im Alkylteil,

beispielsweise die Methoxycarbonyl-, Ethoxycarbonyl-, n-Propoxycarbonyl-, Isopropoxycarbonyl-, n-Butoxycarbonyl-, 1-Methylpropoxycarbonyl-, 2-Methylpropoxy-carbonyl- oder tert.Butyloxycarbonylgruppe,

die Allyloxycarbonyl-, 2,2,2-Trichlor-(1,1-dimethylethoxy)carbonyl- oder 9-Fluorenylmethoxycarbonyl-Gruppe oder die Formyl-, Acetyl- oder Trifluoracetylgruppe.

Die vorliegende Erfindung umfasst auch die einzelnen diastereomeren Antipodenpaare der allgemeinen Formel (I), die zugehörigen Enantiomeren sowie Gemische der unter die allgemeine Formel (I) fallenden Diastereomeren und Enantiomeren.

Besonders bevorzugt sind die unter die allgemeine Formel (I) fallende Racemate und Enantiomeren, die hinsichtlich des Cyclopropanrings trans-konfiguriert sind.

Die Verbindungen der allgemeinen Formel (I) weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Bevorzugte Verbindungen der obigen allgemeinen Formel I sind diejenigen, in denen

R einen einfach oder zweifach ungesättigten 5- bis 7-gliedrigen Aza-, Diaza-, Triaza- oder Thiaza-Heterocyclus,

wobei die vorstehend erwähnten Heterocyclen über ein Kohlenstoff- oder Stickstoffatom verknüpft sein und eine oder zwei Carbonylgruppen benachbart zu einem Stickstoffatom enthalten können,

an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppe substituiert sein können,

und wobei eine olefinische Doppelbindung eines der vorstehend erwähnten ungesättigten Heterocyclen mit einem Benzol-, Pyridin-, Diazin- oder Chinolin-Ring kondensiert sein kann,

wobei die in R enthaltenen Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppen sowie benzo-, pyrido- und diazinokondensierten Heterocyclen im Kohlenstoffgerüst zusätzlich durch Fluor-, Chlor- oder Bromatome, durch Alkyl-, Dialkylaminoalkoxy-, Nitro-, Trifluormethyl-, Alkoxycarbonyl-, Alkoxycarbonylalkyl-, Alkoxycarbonylalkoxy-, Hydroxycarbonylalkoxy-, Carboxy-, Carboxyalkyl-, Dialkylaminoalkyl-, Hydroxy-, Amino-, Acetylamino-, Propionylamino-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, Aminocarbonylamino-, Aminocarbonylaminoalkyl-, Alkylaminocarbonylamino-, Alkanoyl- oder Trifluormethoxy-Gruppen,

durch 5- bis 7-gliedrige Alkyleniminogruppen, in denen eine Methylengruppe in 3-, 4- oder 5-Position durch ein Sauerstoffatom oder eine Methyliminogruppe ersetzt sein kann,

durch Alkoxygruppen, die in ω-Stellung durch einen 5- bis 7-gliedrigen Heteroalicyclus substituiert sein können, wobei der Heteroalicyclus über ein Kohlenstoff- oder Stickstoffatom verknüpft ist und ein oder zwei nicht direkt aneinander gebundene Heteroatome ausgewählt aus Sauerstoff und Stickstoff enthält,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenyl-, 1-Naphthyl- oder 2-Naphthylgruppe,

wobei die vorstehend erwähnten aromatischen Reste durch Fluor-, Chlor- oder Bromatome, durch verzweigte oder unverzweigte Alkylgruppen, Alkoxy-, Trifluormethyl-, Nitro-, Hydroxy-, Amino- oder Acetylaminogruppen mono-, di- oder trisubstituiert und die Substituenten gleich oder verschieden sein können,

und wobei alle vorstehend genannten Alkyl- und Alkoxygruppen sowie die innerhalb der anderen genannten Reste vorhandenen Alkyl- oder Alkylenteile, sofern nichts anderes angegeben ist, 1 bis 4 Kohlenstoffatome umfassen können, darstellen,

deren Tautomere, deren Diastereomere, deren Enantiomere und deren Salze.

Besonders bevorzugte Verbindungen der obigen allgemeinen Formel I sind diejenigen, in denen

R einen einfach ungesättigten 5- bis 7-gliedrigen Diaza- oder Triaza-Heterocyclus,

wobei die vorstehend erwähnten Heterocyclen über ein Stickstoffatom verknüpft sind,

eine Carbonylgruppe benachbart zu einem Stickstoffatom enthalten und

an einem Kohlenstoffatom durch eine Phenyl-Gruppe substituiert oder

eine olefinische Doppelbindung eines der vorstehend erwähnten ungesättigten Heterocyclen mit einem Benzol-, Pyridin- oder Chinolin-Ring kondensiert sein kann,

und wobei die in R enthaltenen Phenyl-Gruppen sowie benzo- und pyrido-kondensierten Heterocyclen im Kohlenstoffgerüst zusätzlich durch Fluor-, Chlor- oder Bromatome, durch Alkyl-, Dialkylaminoalkoxy-, Nitro-, Trifluormethyl-, Alkoxycarbonyl-, Alkoxycarbonylalkoxy-, Hydroxycarbonylalkoxy-, Carboxy-, Hydroxy-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, Alkanoyl- oder Trifluormethoxy-Gruppen,

durch 5- bis 7-gliedrige Alkyleniminogruppen, in denen eine Methylengruppe in 3- oder 4-Position durch ein Sauerstoffatom oder eine Methyliminogruppe ersetzt sein kann, beispielsweise 1-Pyrrolidinyl-, 1-Piperidinyl-, 4-Methyl-1-piperazinyl-, 4-Methyl-1,4-diazacyclohept-1-yl- oder 4-Morpholinylgruppen,

durch Alkoxygruppen, die in ω-Stellung durch einen 5- oder 6-gliedrigen Heteroalicyclus substituiert sein können, wobei der Heteroalicyclus über ein Kohlenstoffatom verknüpft ist und in 2- und 2'-Stellung je ein Sauerstoffatom enthält oder über ein Kohlenstoff- oder Stickstoffatom verknüpft ist und ein oder zwei nicht direkt aneinander gebundene Stickstoffatome oder ein Sauerstoff- und ein Stickstoffatom, die durch mindestens eine Methylengruppe voneinander getrennt sind, enthält, beispielsweise Methoxy-, Ethoxy-, Propoxy-, 2,5-Dioxacyclopentylmethoxy-, 2,6-Dioxacyclohexylmethoxy-, 2-(1-Pyrrolidinyl)ethoxy-, 2-(1-Piperidinyl)ethoxy-, 2-(4-Methyl-1-piperazinyl)ethoxy- oder 2-(4-Morpholinyl)ethoxy-Gruppen,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenylgruppe,

die durch Fluor-, Chlor- oder Bromatome, durch Alkoxy-, Trifluormethyl-, Nitro-, Hydroxy- oder Aminogruppen mono-, di- oder trisubstituiert sein kann, wobei die Substituenten gleich oder verschieden sein können,

wobei alle vorstehend genannten Alkyl- und Alkoxygruppen sowie die innerhalb der anderen genannten Reste vorhandenen Alkyl- oder Alkylenteile, sofern nichts anderes angegeben ist, 1 bis 3 Kohlenstoffatome umfassen können, darstellen,

deren Tautomere, deren Diastereomere, deren Enantiomere und deren Salze.

Ganz besonders bevorzugte Verbindungen der obigen allgemeinen Formel (I) sind diejenigen, in denen

R eine 3,4-Dihydro-2(1H)-oxochinazolin-3-yl-, 1,3-Dihydro-4-phenyl-2H-2-oxoimidazol-1-yl-, 2,4-Dihydro-5-phenyl-3(3H)-oxo-1,2,4-triazol-2-yl-, 3,4-Dihydro-2(1H)-oxopyrido[4,3-d]pyrimidin-3-yl-, 3,4-Dihydro-2(1H)-oxopyrido[3,4-d]pyrimidin-3-yl- oder 1,3-Dihydro-2(2H)-oxoimidazo[4,5-c]chinolin-3-yl-Gruppe,

wobei die vorstehend erwähnten mono- und bicyclischen Heterocyclen im Kohlenstoffgerüst durch Fluor-, Chlor- oder Bromatome mono- oder disubstituiert oder durch eine 4-Methyl-1-piperazinyl-, 2,5-Dioxacyclopentylmethoxy-, Methoxy-, 2-(4-Morpholinyl)ethoxy-, 2-Dimethylaminoethoxy-, 3-Dimethylaminopropoxy-, Methoxycarbonylmethoxy-, Hydroxycarbonylmethoxy-, Nitro-, Trifluormethyl-, Methoxycarbonyl-, Carboxy-, Hydroxy-, Aminocarbonyl-, Diethylaminocarbonyl-, [N-(2-Dimethylaminoethyl)-N-methylamino]carbonyl-, [(Methoxycarbonylmethyl)amino]carbonyl- oder [(Hydroxycarbonylmethyl)amino]carbonyl-Gruppe monosubstituiert sein können,

und $R^1$ eine Phenylgruppe,

die durch Fluor-, Chlor- oder Bromatome, durch Hydroxy- oder Aminogruppen mono-, di- oder trisubstituiert sein kann, wobei die Substituenten gleich oder verschieden sein können, beispielsweise die 4-Chlorphenyl-., 4-Amino-3,5-dibromphenyl- oder 3,5-Dibrom-4-Hydroxyphenylgruppe,

darstellen,

deren Tautomere, deren Diastereomere, deren Enantiomere und deren Salze.

Als ganz besonders bevorzugte Verbindungen seien beispielsweise folgende genannt:

(1) cis-3-{1-[2-(4-Chlorbenzoyl)-cyclopropancarbonyl]-4-piperidinyl}-3,4-dihydro-2(1H)-chinazolinon

(2) trans-1-{1-[2-(4-Amino-3,5-dibrombenzoyl)-cyclopropancarbonyl]-4-piperidinyl}-1,3-dihydro-4-(3-methoxyphenyl)-2(2H)-imidazolon

(3) trans-3-{1-[2-(4-Amino-3,5-dibrombenzoyl)-cyclopropancarbonyl]-4-piperidinyl}-3,4-dihydro-2(1H)-chinazolinon

(4) trans-3-{1-[2-(4-Amino-3,5-dibrombenzoyl)-cyclopropancarbonyl]-4-piperidinyl}-6-brom-3,4-dihydro-2(1H)-chinazolinon

(5) trans-1-{1-[2-(4-Amino-3,5-dibrombenzoyl)-cyclopropancarbonyl]-4-piperidinyl}-1,3-dihydro-4-phenyl-2(2H)-imidazolon

(6) trans-1-{1-[2-(4-Amino-3,5-dibrombenzoyl)-cyclopropancarbonyl]-4-piperidinyl}-1,3-dihydro-4-[3-(trifluormethyl)phenyl]-2(2H)-imidazolon

(7) trans-1-{1-[2-(4-Amino-3,5-dibrombenzoyl)-cyclopropancarbonyl]-4-piperidinyl}-1,3-dihydro-4-(3-hydroxyphenyl)-2(2H)-imidazolon

(8) trans-3-{1-[2-(4-Amino-3,5-dibrombenzoyl)-cyclopropancarbonyl]-4-piperidinyl}-3,4-dihydro-6-hydroxy-2(1H)-chinazolinon

(9) trans-3-{1-[2-(4-Amino-3,5-dibrombenzoyl)-cyclopropancarbonyl]-4-piperidinyl}-3,4-dihydro-6-[(1,3-dioxolan-2-yl)methoxy]-2(1H)-chinazolinon

(10) trans-1-{1-[2-(4-Amino-3,5-dibrombenzoyl)-cyclopropancarbonyl]-4-piperidinyl}-4-(3,4-dichlorphenyl)-1,3-dihydro-2(2H)-imidazolon

(11) trans-3-{1-[2-(4-Amino-3,5-dibrombenzoyl)-cyclopropancarbonyl]-4-piperidinyl}-3,4-dihydro-2(1H)-pyrido [4,3-d]pyrimidinon

(12) trans-1-{1-[2-(4-Amino-3,5-dibrombenzoyl)-cyclopropancarbonyl]-4-piperidinyl}-1,3-dihydro-4-(2-methoxyphenyl)-2(2H)-imidazolon

(13) trans-1-{1-[2-(4-Amino-3,5-dibrombenzoyl)-cyclopropancarbonyl]-4-piperidinyl}-4-(3-chlorphenyl)-1,3-dihydro-2(2H)-imidazolon

(14) trans-1-{1-[2-(4-Amino-3,5-dibrombenzoyl)-cyclopropancarbonyl]-4-piperidinyl}-1,3-dihydro-4-(3-nitrophenyl)-2(2H)-imidazolon

(15) trans-3-{1-[2-(4-Amino-3,5-dibrombenzoyl)-cyclopropancarbonyl]-4-piperidinyl}-3,4-dihydro-2(1H)-pyrido [3,4-d]pyrimidinon

(16) trans-3-{1-[2-(4-Amino-3,5-dibrombenzoyl)-cyclopropancarbonyl]-4-piperidinyl}-3,4-dihydro-6-[2-(dimethylamino)ethoxy]-2(1H)-chinazolinon

(17) trans-3-{1-[2-(4-Amino-3,5-dibrombenzoyl)-cyclopropancarbonyl]-4-piperidinyl}-3,4-dihydro-6-(4-methyl-1-piperazinyl)-2(1H)-chinazolinon

(18) trans-1-{1-[2-(4-Amino-3,5-dibrombenzoyl)-cyclopropancarbonyl]-4-piperidinyl}-1,3-dihydro-4-[2-(trifluormethyl)phenyl]-2 (2H)-imidazolon

(19) trans-3-{1-[2-(4-Amino-3,5-dibrombenzoyl)-cyclopropancarbonyl]-4-piperidinyl}-3,4-dihydro-6-[3-(dimethylamino)propoxy]-2(1H)-chinazolinon

(20) trans-3-{1-[2-(3,5-Dibrom-4-hydroxybenzoyl)-cyclopropancarbonyl]-4-piperidinyl}-3,4-dihydro-2(1H)-chinazolinon

(21) trans-1-{1-[2-(3,5-Dibrom-4-hydroxybenzoyl)-cyclopropancarbonyl]-4-piperidinyl}-1,3-dihydro-4-phenyl-2 (2H)-imidazolon

(22) trans-3-{1-[2-(4-Amino-3,5-dibrombenzoyl)-cyclopropancarbonyl]-4-piperidinyl}-3,4-dihydro-6-(methoxycarbonylmethoxy)-2(1H)-chinazolinon

(23) trans-3-{1-[2-(4-Amino-3,5-dibrombenzoyl)-cyclopropancarbonyl]-4-piperidinyl}-3,4-dihydro-6-(hydroxycarbonylmethoxy)-2(1H)-chinazolinon

(24)trans-3-{1-[2-(4-Amino-3,5-dibrombenzoyl)-cyclopropancarbonyl]-4-piperidinyl}-3,4-dihydro-6-[2-(4-morpholinyl)ethoxy]-2(1H)-chinazolinon

(25) trans-3-{1-[2-(4-Amino-3,5-dibrombenzoyl)-cyclopropancarbonyl]-4-piperidinyl}-3,4-dihydro-7-methoxy-2(1H)-chinazolinon

(26) trans-3-{1-[2-(4-Amino-3,5-dibrombenzoyl)-cyclopropancarbonyl]-4-piperidinyl}-3,4-dihydro-7-(methoxycarbonylmethoxy)-2(1H)-chinazolinon

(27) trans-3-{1-[2-(4-Amino-3,5-dibrombenzoyl)-cyclopropancarbonyl]-4-piperidinyl}-7-carboxy-3,4-dihydro-2(1H)-chinazolinon

(28) trans-3-{1-[2-(4-Amino-3,5-dibrombenzoyl)-cyclopropancarbonyl]-4-piperidinyl}-7-methoxycarbonyl-3,4-dihydro-2(1H)-chinazolinon

(29) trans-3-{1-[2-(4-Amino-3,5-dibrombenzoyl)-cyclopropancarbonyl]-4-piperidinyl}-1,3-dihydro-2(2H)-imidazo[4,5-c]chinolinon

(30) trans-3-{1-[2-(4-Amino-3,5-dibrombenzoyl)-cyclopropancarbonyl]-4-piperidinyl}-3,4-dihydro-7-{[(methoxycarbonylmethyl)amino]carbonyl}-2(1H)-chinazolinon

(31) trans-3-{1-[2-(4-Amino-3,5-dibrombenzoyl)-cyclopropancarbonyl]-4-piperidinyl}-3,4-dihydro-7-{[N-(2-dimethylaminoethyl)-N-methylamino]carbonyl}-2(1H)-chinazolinon

(32) trans-3-{1-[2-(4-Amino-3,5-dibrombenzoyl)-cyclopropancarbonyl]-4-piperidinyl}-7-diethylaminocarbonyl-3,4-dihydro-2(1H)-chinazolinon

(33) trans-7-Aminocarbonyl-3-{1-[2-(4-amino-3,5-dibrombenzoyl)-cyclopropancarbonyl]-4-piperidinyl}-3,4-dihydro-2(1H)-chinazolinon

(34) trans-3-{1-[2-(4-Amino-3,5-dibrombenzoyl)-cyclopropancarbonyl]-4-piperidinyl}-3,4-dihydro-7-{[(hydroxycarbonylmethyl)amino]carbonyl}-2(1H)-chinazolinon

(35) trans-1-{1-[2-(4-Amino-3,5-dibrombenzoyl)-cyclopropancarbonyl]-4-piperidinyl}-2,4-dihydro-5-phenyl-3(3H)-1,2,4-triazolon,

insbesondere die vorstehend erwähnten Verbindungen (2), (3), (5), (7), (8), (9), (13), (19), (22), (23) und (35), sowie deren Salze.

Die Verbindungen der allgemeinen Formel I werden nach prinzipiell bekannten Methoden hergestellt. Die folgenden Verfahren haben sich zur Herstellung der erfindungsgemäßen Verbindungen der allgemeinen Formel I besonders bewährt:

a) Kupplung einer Carbonsäure der allgemeinen Formel

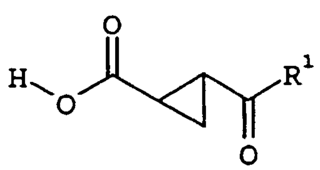

(II),

in der
$R^1$ wie eingangs erwähnt definiert ist,
mit einer Verbindung der allgemeinen Formel

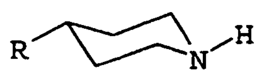

(III),

in der
R wie eingangs erwähnt definiert ist.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylaminopropyl)-carbodiimid, O-(1H-Benzotriazol-1-yl)-N,N,N',N'-tetramethyluroniumhexafluorophosphat (HBTU) oder -tetrafluoroborat (TBTU) oder 1H-Benzotriazol-1-yl-oxy-tris-(dimethylamino)-phosphoniumhexafluorophosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann eine eventuelle Konfigurationsänderung gewünschtenfalls zusätzlich unterdrückt bzw. die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), N-Methylpyrrolidon (NMP) oder Gemischen aus diesen und bei Temperaturen zwischen -30 und +30°C, bevorzugt -20 und +25°C, durchgeführt. Sofern erforderlich, wird als zusätzliche Hilfsbase N-Ethyl-diisopropylamin (DIEA) (Hünig-Base) bevorzugt.

Als weiteres Kupplungsverfahren zur Synthese von Verbindungen der allgemeinen Formel I wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel II und dem Kohlensäuremonoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwischen -20 und +25°C, bevorzugt 0 und +25°C.

b) Kupplung einer Verbindung der allgemeinen Formel

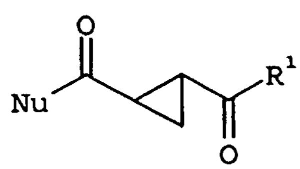

(IV),

in der
$R^1$ wie eingangs erwähnt definiert ist und
Nu eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1H-Imidazol-1-yl-, eine gegebenenfalls durch 1 oder 2 Methylgruppen im Kohlenstoffgerüst substituierte 1H-Pyrazol-1-yl-, 1H-1,2,4-Triazol-1-yl-, 1H-1,2,3-Triazol-1-yl- oder 1H-1,2,3,4-Tetrazol-1-yl-Gruppe, eine Vinyl-, Propargyl-, p-Nitrophenyl-, 2,4-Dinitrophenyl-, Trichlorphenyl-, Pentachlorphenyl-, Pentafluorphenyl-, Pyranyl- oder Pyridinyl-, Dimethylaminyloxy-, 2(1H)-Oxopyridin-1-yloxy-, 2,5-Dioxopyrrolidin-1-yloxy-, Phthalimidyloxy-, 1H-Benzotriazol-1-yloxy- oder Azidgruppe bedeutet,
mit einer Verbindung der allgemeinen Formel

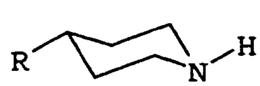

(III),

in der
R wie eingangs erwähnt definiert ist.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyl-diisopropylamin, N-Ethyl-dicyclohexylamin, 1,4-Diazabicyclo[2,2,2]octan oder 1,8-Diazabicyclo[5,4,0]undec-7-en, als Lösemittel beispielsweise Dichlormethan, Tetrahydrofuran, 1,4-Dioxan, Acetonitril, Dimethylformamid, Dimethylacetamid, N-Methyl-pyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

c) Cyclopropanierung einer Verbindung der allgemeinen Formel

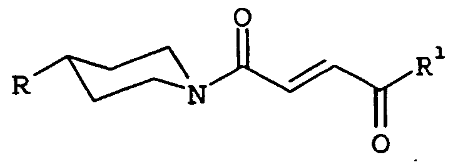

(V),

in der
R und $R^1$ wie eingangs erwähnt definiert sind.

Die Cyclopropanierung kann katalytisch mit Diazomethan erfolgen, wobei Ausgangsverbindungen der Formel (V) verwendet werden, in denen die olefinische Doppelbindung bevorzugt (E)-konfiguriert ist. Die Reaktion wird bei Temperaturen zwischen 0°C und + 50°C, bevorzugt bei Zimmertemperatur durchgeführt. Als Katalysatoren werden Palladium(II)-Verbindungen bevorzugt, beispielsweise $PdCl_2(PhCN)_2$ oder Palladium(II)-acetat $Pd_3(OAc)_6$. Als Lösemittel kommen inerte Ether, beispielsweise Diethylether, Kohlenwasserstoffe und ganz bevorzugt chlorierte Kohlenwasserstoffe, wie Dichlormethan oder 1,2-Dichlorethan, oder auch Gemische der genannten Lösemittel in Betracht (siehe auch: H. Abdallah, R. Green und R. Carrie, Tetrahedron Letters 23, 503-506 (1982)). Die Cyclopropanierung (E)-konfigurierter Verbindungen der allgemeinen Formel V kann durch Verwendung der von A. Pfaltz, Acc. Chem. Res. 26, 339-345 (1993), beschriebenen Semicorrin-Cupfer-Katalysatoren unter Erzielung eines hohen Enantiomeren-Überschusses auch asymmetrisch gestaltet werden. Das erforderliche Diazomethan kann auch in situ erzeugt werden, indem man N-Methyl-N-nitrosoharnstoff portionsweise zu einer Mischung aus einem Alken der allgemeinen Formel (V), dem Palladium-Katalysator, dem organischen Lösemittel und 40- bis 50-prozentiger wässeriger Kaliumhydroxid-Lösung gibt; bei dieser Arbeitsweise werden in der Regel höchstens 2 Mol N-Methyl-N-nitrosoharnstoff pro Mol des Alkens der allgemeinen Formel (V) benötigt.

Ferner kann die Cyclopropanierung von Alkenen der allgemeinen Formel (V), in der die olefinische Doppelbindung beliebig orientiert sein kann, wobei die (E)-Konfiguration aber bevorzugt wird, in Analogie zur sogenannten Simmons-Smith-Reaktion mit Diiodmethan und dem Zink-Cupfer-Paar (s. auch: Simmons, Cairns, Vladuchik und Hoiness, Org. React. **20,** 1–131 (1973); Furukawa und Kawabata, Adv. Organomet. Chem. **12,** 83–134 (1974)) oder dem Zink-Silber-Paar (s. auch: J. M. Denis, C. Girard und J. M. Conia, Synthesis **1972**, 549) erfolgen. Das Zink-Cupfer-Paar kann nach mehreren Varianten erzeugt werden (siehe z. B.: Shank und Shechter, J. Org. Chem. **24,** 1525 (1959); LeGoff, J. Org. Chem. **29,** 2048 (1964)), von denen das Erhitzen von Zinkstaub mit Cupfer(I)-chlorid in Diethylether und unter Stickstoff (Rawson und Harrison, J. Org. Chem. **35,** 2057 (1970)) besonders geeignet ist. Die Reaktion gelingt auch mit nicht aktiviertem Zink im Ultraschallbad (siehe auch: Repi und Vogt, Tetrahedron Letters 23, 2729 (1982); Repi , Lee und Giger, Org. Prep. Proced. Int. 16, 25 (1984). Die das Alken der allgemeinen Formel (V) angreifende Spezies ist eine intermediär auftretende Organozinkverbindung, Bis-(iodmethyl)-zink (siehe auch: Georg Wittig und Frank Wingler, Chem. Ber. **97,** 2146 (1964)) oder das Addukt $(ICH_2)_2Zn{\cdot}ZnI_2$ (Blanchard und Simmons, J. Am. Chem. Soc. **86,** 1337 (1964)), deren Lösungen für physikochemische Untersuchungen hinreichend stabil sind. Die Cyclopropanierung erfolgt stereospezifisch syn. Die Reaktivität des Reagens kann durch Zusatz einer Lewis-Säure, beispielsweise von Nickel(II)-bromid, gesteigert werden (s. auch: H. Kanai et al., Bull. Chem. Soc. Jap. **56**, 1025–1029 (1983), Synthesis **1984,** 987), das erforderliche Diiodmethan auch in situ aus Dibrommethan und Natriumiodid erzeugt werden. In einer weiteren Variante der Cyclopropanierung wird das Substrat der allgemeinen Formel (V) mit Diiodmethan oder einem anderen Dihalomethan und Diethylzink umgesetzt (s. auch: Furukawa, Kawabata und Nishimura, Tetrahedron **24,** 53 (1968), Tetrahedron Letters 1968, 3495; Nishimura, Kawabata und Furukawa, Tetrahedron **25,** 2647 (1969); Miyano und

Hashimoto, Bull. Chem. Soc. Jpn. **46**, 892 (1973); Friedrich und Biresaw, J. Org. Chem. 47, 1615 (1982)). Schließlich kann das erforderliche Reagens auch aus Dihalomethanen und Cupfer erzeugt werden (Kawabata, Kamemura und Naka, J. Am. Chem. Soc. **101**, 2139 (1979); Kawabata, Tanimoto und Fujiwara, Tetrahedron **35,** 1919 (1979)). Die Cyclopropanierung wird bei Temperaturen zwischen 0°C und +70°C, bevorzugt bei Zimmertemperatur, und unter Verwendung von etherischen Lösemitteln, beispielsweise Diethylether oder Tetrahydrofuran, durchgeführt.

Die Cyclopropanierung eines Alkens der allgemeinen Formel (V), in der die olefinische Doppelbindung beliebig orientiert sein kann, wobei die (E)-Konfiguration aber bevorzugt wird, kann auch mit dem Dimethyloxosulfoniummethylid der Formel

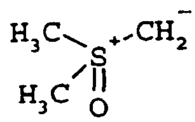 (VI),

oder einem Dialkylamino-oxosulfoniummethylid der allgemeinen Formel

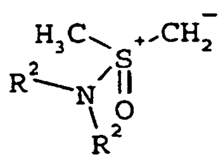 (VII),

in der
$R^2$ die Methyl- oder Ethylgruppe bedeutet, durchgeführt werden.

Die Umsetzung wird in dipolaren, aprotischen Lösemitteln, bevorzugt in Dimethylsulfoxid, und bei Temperaturen zwischen +10 und +80°C, bevorzugt +20 und +60°C, durchgeführt. Die Oxosulfoniumylide VI und VII können als solche eingesetzt werden, werden jedoch auch in situ aus dem Trimethyloxosulfoniumiodid der Formel

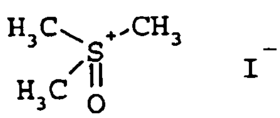 (VIII),

durch Einwirkung von Methansulfinylmethylnatrium (siehe auch: E. J. Corey und M. Chaykowsky, J. Am. Chem. Soc. **87**, 1353 (1965), Org. Syn. **49**, 78 (1969); H. Schmidbauer und W. Tronich, Tetrahedron Letters **1968,** 5335) bzw. aus einem Dialkylaminooxosulfoniumiodid der allgemeinen Formel

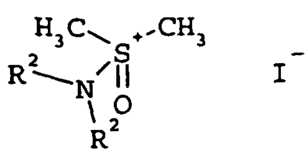 (IX),

in der
$R^2$ wie vorstehend definiert ist, durch Einwirkung von Natriumhydrid (siehe auch: C. R. Johnson, E. R. Janiga und M. Haake, J. Am. Chem. Soc. **90**, 3890 (1968); C. R. Johnson und C. W. Schroeck, J. Am. Chem. Soc. **90**, 6852 (1968); C. R. Johnson und G. F. Katekar, J. Am. Chem. Soc. **92**, 5753 (1970); C. R. Johnson, M. Haake und C. W. Schroeck, J. Am. Chem. Soc. **92**, 6594 (1970); C. R. Johnson und P. E. Rogers, J. Org. Chem. **38**, 1793 (1973) in Dimethylsulfoxid erzeugt. Ylide der allgemeinen Formel VII sind auch in optisch aktiver Form erhältlich und eignen sich damit zur asymmetrischen Synthese von Verbindungen der allgemeinen Formel (I).

d) Zur Herstellung einer Verbindung der allgemeinen Formel I, in der mindestens einer der Reste R und $R^1$ eine

oder mehrere Carboxygruppen enthält:

Alkalische Verseifung eines Carbonsäureesters der allgemeinen Formel

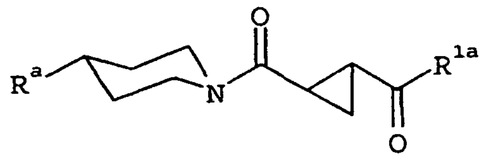

(Ia),

in der

$R^a$ und $R^{1a}$ mit der Maßgabe, daß mindestens einer dieser Reste eine oder mehrere Alkoxycarbonylgruppen enthält, die eingangs für R bzw. $R^1$ angegebenen Bedeutungen besitzen,

und gewünschtenfalls anschließende Behandlung mit verdünnten organischen oder anorganischen Säuren zwecks Freisetzung der zugrundeliegenden Carbonsäuren aus ihren zunächst entstandenen Salzen.

Zur alkalischen Verseifung der Ester der allgemeinen Formel (Ia) werden Lithiumhydroxid, Natriumhydroxid und Kaliumhydroxid bevorzugt; geeignet sind aber auch andere Alkalihydroxide, beispielsweise Cäsiumhydroxid, oder Erdalkalihydroxide, beispielsweise Bariumhydroxid, oder auch Tetralkylammoniumhydroxide. Die Durchführung erfolgt in wässeriger Lösung und vorteilhaft unter Zusatz von mit Wasser mischbaren Cosolvenzien, bevorzugt von Alkoholen, wie Methanol, Ethanol oder 2-Ethoxyethanol, oder von Ethern, wie Tetrahydrofuran oder 1,4-Dioxan. Zur alkalischen Verseifung geeignete Temperaturen liegen zwischen -10°C und der Siedetemperatur des verwendeten Wasser-Lösemittel-Gemischs, bevorzugt wird jedoch Zimmertemperatur. Zur Freisetzung der zugrundeliegenden Carbonsäuren aus ihren zunächst entstandenen Salzen eignen sich verdünnte wässerige organische oder anorganische Säuren, z. B. Essigsäure, Oxalsäure, Methansulfonsäure, Salzsäure, Schwefelsäure, Phosphorsäure.

e) Zur Herstellung einer Verbindung der allgemeinen Formel I, in der

der Rest R im Kohlenstoffgerüst durch eine Aminocarbonyl-, Alkylaminocarbonyl-, Dialkylaminocarbonyl-, [N-Alkyl-N-(dialkylaminoalkyl)amino]carbonyl-, Hydroxycarbonylalkylaminocarbonyl-, Alkoxycarbonylalkylaminocarbonyl-, (4-Morpholinyl)carbonyl-, (1-Pyrrolidinyl)carbonyl-, (1-Piperidinyl)carbonyl-, (Hexahydro-1-azepinyl)carbonyl- oder (4-Methyl-1-piperazinyl)carbonyl-Gruppe gleichartig mono-, di- oder trisubstituiert ist:

Kupplung einer Verbindung der allgemeinen Formel

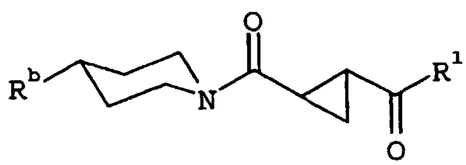

(Ib),

in der

$R^1$ wie eingangs erwähnt definiert ist und

der Rest $R^b$ mit der Maßgabe, daß er im Kohlenstoffgerüst durch die Carboxy-Gruppe mono-, di- oder trisubstituiert ist, die eingangs für R angegebenen Bedeutungen hat,

mit Ammoniak, Alkylaminen, N-Alkyl-N-(dialkylaminoalkyl)aminen, Hydroxycarbonylalkylaminen, Alkoxycarbonylalkylaminen oder Dialkylaminen, beispielsweise 1-Methylpiperazin, Morpholin, Pyrrolidin, Piperidin oder Hexahydroazepin.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylaminopropyl)-carbodiimid, O-(1H-Benzotriazol-1-yl)-N,N-N',N'-tetramethyluroniumhexafluorophosphat (HBTU) oder -tetrafluoroborat (TBTU) oder 1H-Benzotriazol-1-yl-oxy-tris-(dimethylamino)phosphoniumhexafluorophosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann eine eventuelle Racemisierung gewünschtenfalls zusätzlich unterdrückt bzw. die Reaktionsgeschwindigkeit gesteigert

werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), N-Methylpyrrolidon (NMP) oder Gemischen aus diesen und bei Temperaturen zwischen -30 und +30°C, bevorzugt -20 und +20°C, durchgeführt. Sofern erforderlich, wird als zusätzliche Hilfsbase N-Ethyl-diisopropylamin (DIEA) (Hünig-Base) bevorzugt.

Als weiteres Kupplungsverfahren zur Synthese von Verbindungen der allgemeinen Formel I wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden, gegebenenfalls $N^2$-geschützten $\alpha$-Aminosäure und dem Kohlensäuremonoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwischen -20 und +20°C, bevorzugt 0 und +20°C.

Die erfindungsgemäßen neuen Cyclopropane der allgemeinen Formel (I) enthalten wenigstens zwei Chiralitätszentren. Mithin treten die Verbindungen in Form zweier diastereomerer Antipodenpaare auf. Die Erfindung umfaßt die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, bzw. optisch aktiven Base, beispielsweise mit (R)-(+)-1-Phenylethylamin, (S)-(-)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösungsmittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, Natronlauge oder Kalilauge neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel I fallender diastereomerer Verbindungen wird auch dadurch erhalten, daß man die oben beschriebenen Synthesen mit jeweils einer geeigneten (R)- bzw. (S)-konfigurierten Reaktionskomponente durchführt.

Die Ausgangsverbindungen der allgemeinen Formel (Ia) und (Ib) lassen sich nach den in dieser Anmeldung beschriebenen Verfahren a) bis c) herstellen. Die zur Synthese der Verbindungen der allgemeinen Formel I erforderlichen Ausgangsmaterialien der allgemeinen Formeln II lassen sich, soweit nicht literaturbekannt, beispielweise aus den entsprechenden Carbonsäureestern, wie den Methyl- oder Ethylestern, durch Verseifung mit wässeriger Lithium-, Natrium- oder Kaliumhydroxid-Lösung und anschließendes Ansäuern mit Salzsäure in Analogie zu dem Fachmann bekannten Verfahren leicht herstellen. Die hierfür benötigten Carbonsäureester sind aus den entsprechenden 4-Aryl- bzw. Hetaryl-4-oxo-2-butensäurestern beispielsweise durch Umsetzung mit Dimethyloxosulfoniummethylid in Analogie zu dem vorstehend unter c) beschriebenen Verfahren erhältlich. 4-Aryl- bzw. Hetaryl-4-oxo-2-butensäureester schließlich sind entweder literaturbekannt oder aus literaturbekannten 4-Aryl- bzw. Hetaryl-4-oxo-2-butensäuren gut zugänglich (siehe auch deutsche Offenlegungsschriften 2 047 806 und 2 103 749).

Sekundäre Amine der allgemeinen Formel III sind entweder bekannt oder lassen sich beispielsweise in Analogie zu in der WO 98/11128 beschriebenen Verfahren synthetisieren.

Ausgangsverbindungen der allgemeinen Formel IV sind aus den Ausgangsverbindungen der allgemeinen Formel II nach gängigen Methoden erhältlich.

Die Ausgangsverbindungen der allgemeinen Formel V sind beispielsweise durch Acylierung von Verbindungen der Formel (III) mit ungesättigten Carbonsäurederivaten leicht herstellbar.

Die erhaltenen Verbindungen der allgemeinen Formel I können, sofern sie geeignete basische Funktionen enthalten, insbesondere für pharmazeutische Anwendungen in ihre physiologisch verträglichen Salze mit anorganischen oder organischen Säuren, übergeführt werden. Als Säuren kommen hierfür beispielsweise Salzsäure, Bromwasserstoffsäure, Phosphorsäure, Salpetersäure, Schwefelsäure, Methansulfonsäure, p-Toluolsulfonsäure, Essigsäure, Fumarsäure, Bernsteinsäure, Milchsäure, Mandelsäure, Äpfelsäure, Zitronensäure, Weinsäure oder Maleinsäure

in Betracht.

Außerdem lassen sich die neuen Verbindungen der Formel (I), falls sie eine saure Funktion, beispielsweise eine Carboxygruppe enthalten, gewünschtenfalls in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Dicyclohexylamin, Äthanolamin, Diäthanolamin und Triäthanolamin in Betracht.

Die neuen Verbindungen der allgemeinen Formel I und deren physiologisch verträglichen Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität von Verbindungen der allgemeinen Formel I zu humanen CGRP-Rezeptoren und ihrer antagonistischen Eigenschaften wurden die folgenden Versuche durchgeführt:

A. Bindungsstudien mit (den humanen CGRP-Rezeptor exprimierenden SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, $NaHCO_3$ 16.2, $MgSO_4$ 0.8, $NaHPO_4$ 1.0, $CaCl_2$ 1.8, D-Glucose 5.5, HEPES 30, pH7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM $MgCl_2$, 1 mM EDTA, pH 7.40), angereichert mit 1% Rinderserum-Albumin und 0.1% Bacitracin, rezentrifugiert und resuspendiert (1 ml/1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM $MgCl_2$, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM $^{125}$I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die Verbindungen der allgemeinen Formel I zeigen in dem beschriebenen Test $IC_{50}$-Werte ≤ 10000 nM.

B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen ($10^{-11}$ bis $10^{-6}$ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die $pA_2$-Werte antagonistisch wirkender Substanzen graphisch ermittelt.

Die Verbindungen der allgemeinen Formel I zeigen in dem beschriebenen in-vitro-Testmodell CGRP-antagonistische Eigenschaften in einem Dosisbereich zwischen $10^{-11}$ bis $10^{-5}$ M.

Auf Grund ihrer pharmakologischen Eigenschaften eignen sich die Verbindungen der allgemeinen Formel I und deren Salze mit physiologisch verträglichen Säuren bzw. Basen somit zur akuten und prophylaktischen Behandlung von Kopfschmerzen, insbesondere Migräne- bzw. Cluster-Kopfschmerz. Weiterhin beeinflussen die Verbindungen der allgemeinen Formel I auch die folgenden Erkrankungen positiv: Nicht-insulinabhängigen Diabetes mellitus ("NIDDM"), cardiovaskuläre Erkrankungen, Morphintoleranz, Erkrankungen der Haut, insbesondere thermische und strahlenbedingte Hautschäden inclusive Sonnenbrand, entzündliche Erkrankungen, z.B. entzündliche Gelenkerkrankungen (Arthritis), entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Die Symptomatik menopausaler, durch Gefäßerweiterung und erhöhten Blutfluß verursachter Hitzewallungen östrogendefizienter Frauen wird durch die CGRP-Antagonisten der vorliegenden Anwendung präventiv und akuttherapeutisch günstig beeinflußt, wobei sich dieser Therapieansatz vor der Hormonsubstitution durch Nebenwirkungsarmut auszeichnet. Darüber hinaus zeigen die Verbindungen der allgemeinen Formel I eine lindernde Wirkung auf Schmerzzustände im allgemeinen.

Die zur Erzielung einer entsprechenden Wirkung erforderliche Dosierung beträgt zweckmäßigerweise bei intravenöser oder subcutaner Gabe 0,001 bis 30 mg/kg Körpergewicht, vorzugsweise 0,01 bis 5 mg/kg Körpergewicht, und bei oraler, nasaler oder inhalativer Gabe 0,01 bis 50 mg/kg Körpergewicht, vorzugsweise 0,1 bis 30 mg/kg Körpergewicht, jeweils 1 bis 3 x täglich.

Hierzu lassen sich die erfindungsgemäß hergestellten Verbindungen der allgemeinen Formel I, gegebenenfalls in Kombination mit anderen Wirksubstanzen, insbesondere mit denen die bei der Behandlung der Migräne eingesetzt werden, mit Antiemetica, Prokinetica, Neuroleptica, Antidepressiva, Neurokinin-Antagonisten, Anticonvulsiva, Histamin-H1-Rezeptorantagonisten, Antimuscarinika, β-Blockern, α-Agonisten und α-Antagonisten, Ergotalkaloiden, schwachen Analgetica, nichtsteroidalen Antiphlogistica, Corticosteroiden, Calcium-Antagonisten, 5-$HT_{1D}$-Agonisten oder anderen Antimigränemitteln, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen einarbeiten.

Für die oben erwähnten Kombinationen kommen somit als weitere Wirksubstanzen beispielsweise Meloxicam, Ergotamin, Dihydroergotamin, Metoclopramid, Domperidon, Diphenhydramin, Cyclizin, Promethazin, Chlorpromazin, Dexamethason, Flunarizin, Dextropropoxyphen, Meperidin, Propranolol, Nadolol, Atenolol, Clonidin, Indoramin, Carbamazepin, Phenytoin, Valproat, Amitryptilin, Lidocain, Diltiazem oder Sumatriptan und andere 5-$HT_{1D}$-, 5-$HT_{1B/D}$- oder 5-$HT_{1F}$-Agonisten wie z.B. Naratriptan, Zolmitriptan, Avitriptan, Rizatriptan und Eletriptan in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der Verbindungen der allgemeinen Formel I als wertvolle Hilfsmittel zur Erzeugung und Reinigung (Affinitätschromatographie) von Antikörpern sowie, nach geeigneter radioaktiver Markierung, beispielsweise durch direkte Markierung mit $^{125}I$ oder $^{131}I$ oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neurotransmitter-Forschung.

Die nachfolgenden Beispiele sollen die Erfindung näher erläutern:

<u>Vorbemerkungen:</u>

Für alle Verbindungen liegen befriedigende Elementaranalysen, IR-, UV-, $^{1}H$-NMR und in der Regel auch Massenspekten vor. Wenn nicht anders angegeben, wurden $R_f$-Werte unter Verwendung von DC-Fertigplatten Kieselgel 60 $F_{254}$ (E. Merck, Darmstadt, Artikel-Nr. 5729) ohne Kammersättigung bestimmt. Falls nähere Angaben zur Konfiguration fehlen, bleibt offen, ob es sich um reine Enantiomere handelt oder ob partielle oder gar völlige Racemisierung eingetreten ist. Zur Chromatographie wurden die folgenden Fließmittel bzw. Fließmittelgemische verwendet:

FM A = Essigsäureethylester/Methanol 100/5 v/v
FM B = Essigsäureethylester/Methanol 80/20 v/v
FM C = Essigsäureethylester/Methanol/konz. Ammoniak 80/20/1 v/v/v
FM D = Dichlormethan/Cyclohexan/Methanol/konz. Ammoniak 70/15/15/2 v/v/v/v
FM E = Essigsäureethylester/Eisessig 99/1 v/v
FM F = Essigsäureethylester/Methanol/Eisessig 90/10/1 v/v/v
FM G = Dichlormethan/Methanol/konz. Ammoniak 90/10/1 v/v/v
FM H = Petrolether/Essigsäureethylester 1/1 v/v
FM I = Dichlormethan/Methanol/Eisessig 90/10/1.5 v/v/v
FM K = Dichlormethan/Isopropanol 9/1 v/v
FM L = Essigsäureethylester/Methanol 9/1 v/v
FM M = Dichlormethan/Methanol/konz. Ammoniak 75/25/5 v/v/v
FM N = Dichlormethan/Essigsäureethylester 1/1 v/v
FM O = Dichlormethan/Methanol 95/5 v/v
FM P = Dichlormethan/Essigsäureethylester/Cyclohexan/Methanol/konz. Ammoniak 60/16/5/5/0.6 v/v/v/v/v

In der Versuchsbeschreibung werden die folgenden Abkürzungen verwendet:

Fp.: Schmelzpunkt
(Z) : (Zersetzung)
DIEA: N,N-Diisopropyl-ethylamin
Boc: (1,1-Dimethylethoxy)carbonyl
TBTU: 2-(1H-Benzotriazol-1-yl)-1,1,3,3-tetramethyluroniumtetrafluoroborat
HOBt: 1-Hydroxybenzotriazol-hydrat
CDT: 1,1'-Carbonyldi(1,2,4-triazol)
THF: Tetrahydrofuran
DMF: Dimethylformamid
EE: Essigsäureethylester
PE: Petrolether
LM: Lösemittel
Lfd. Nr.: Laufende Nummer

Die Bedeutung der in den Beispielen verwendeten, aus Buchstaben und Zahlen zusammengesetzten Symbole ergibt sich aus der folgenden Übersicht:

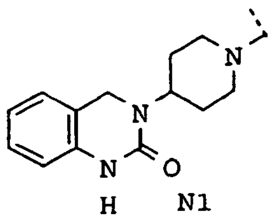
N1

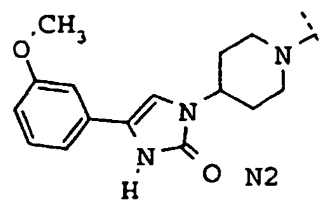
N2

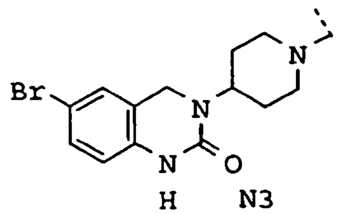
N3

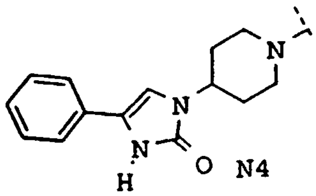
N4

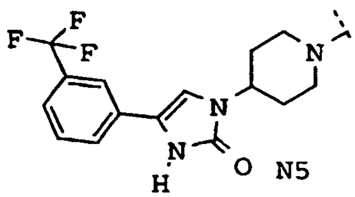
N5

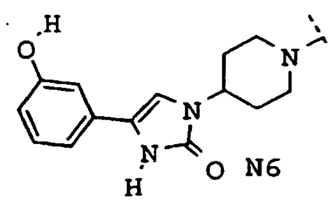
N6

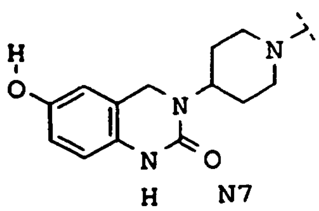
N7

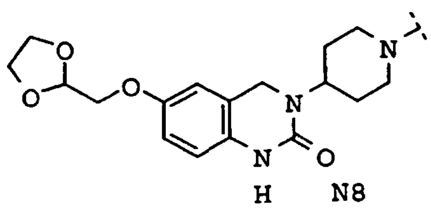
N8

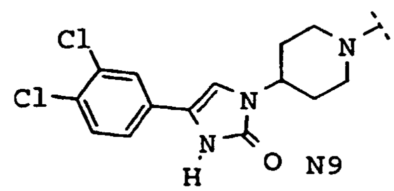
N9

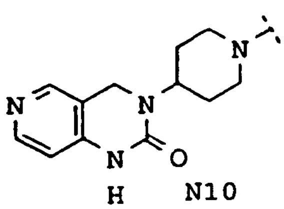
N10

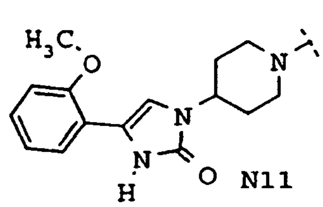
N11

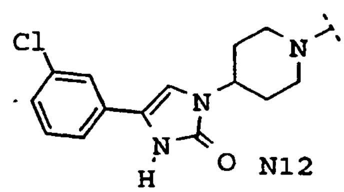
N12

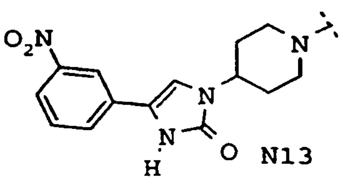
N13

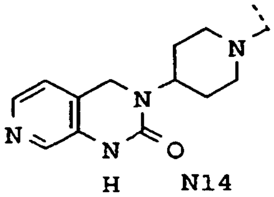
N14

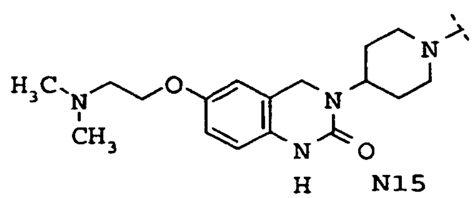
N15

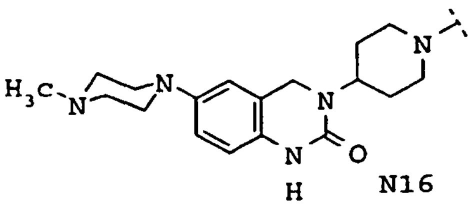
N16

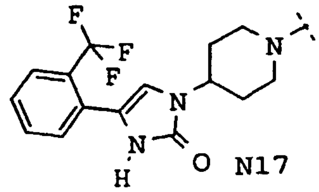
N17

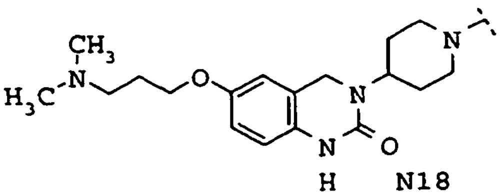
N18

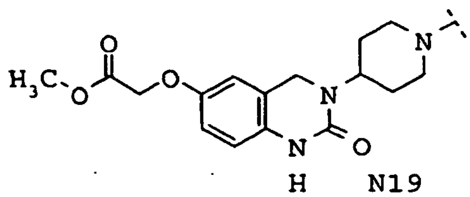
N19

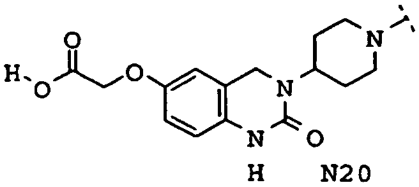
N20

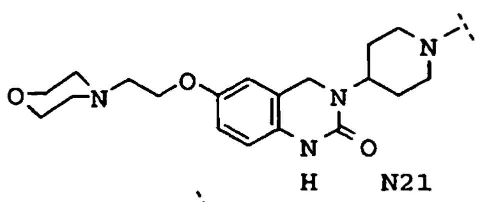
N21

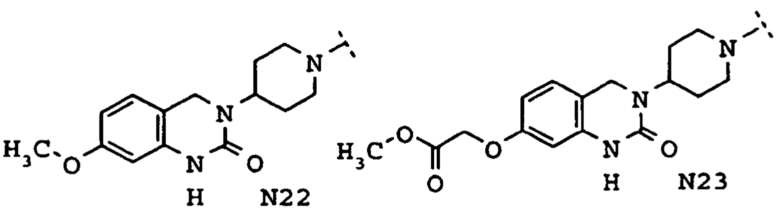
N22

N23

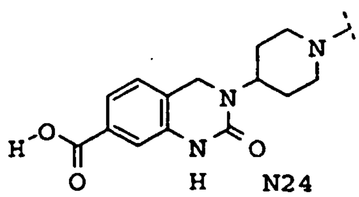
N24

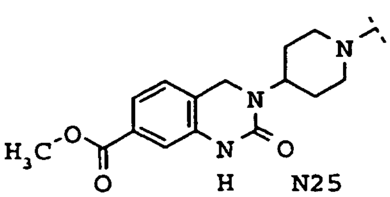
N25

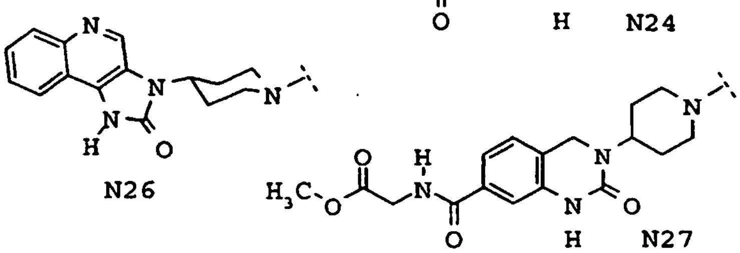
N26

N27

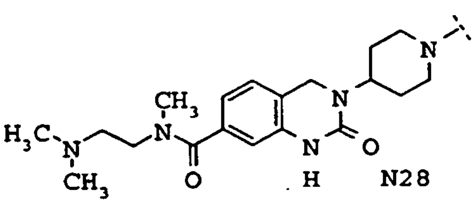
N28

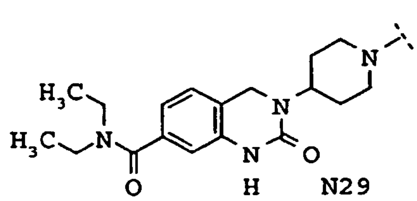
N29

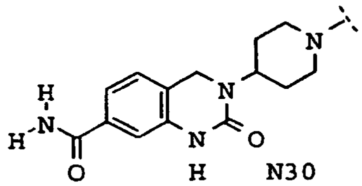
N30

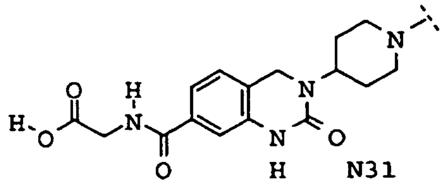
N31

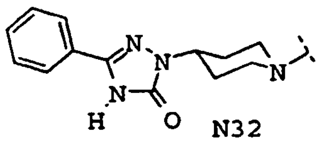
N32

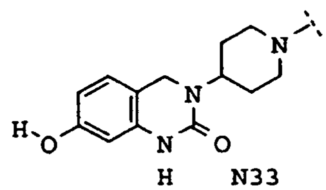
N33

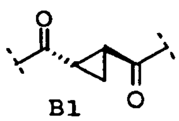
B1

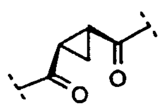
B2

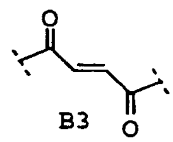
B3

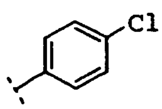
C1

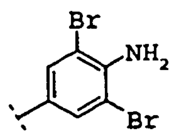
C2

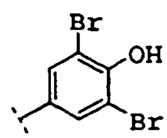
C3

**A. Herstellung von Zwischenverbindungen**

Beispiel A1

cis-2-(4-Chlorbenzoyl)-cyclopropancarbonsäure

Zu der Mischung aus 60.0 g(0.45 Mol) wasserfreiem Aluminiumchlorid und 9 ml (0.115 Mol) wasserfreiem Dimethylformamid tropfte man unter Einhaltung einer Reaktionstemperatur von 60 bis 70°C nacheinander 4.5 g (0.040 Mol) Chlorbenzol und 5.0 g (0.0446 Mol) 1,2-Cyclopropandicarbonsäureanhydrid und hielt das Gemisch anschließend noch 1 Stunde bei 70°C. Das erkaltete Reaktionsgemisch wurde in ein Gemisch aus 500 g gestoßenem Eis und 60 ml konz. Salzsäure eingerührt, der Niederschlag abgenutscht, gründlich mit Wasser gewaschen und im Vakuumtrokkenschrank über Siccapent bei einer Temperatur von 50°C getrocknet. Man erhielt 7.8 g (87 % der Theorie) an farblosen Kristallen vom Fp. 150-153°C.

Beispiel A2

trans-2-(4-Amino-3,5-dibrombenzoyl)-cyclopropancarbonsäure

Hergestellt analog Beispiel 2 aus trans-2-(4-Amino-3,5-dibrombenzoyl)-cyclopropancarbonsäuremethylester durch Verseifung mit Lithiumhydroxid-hydrat in einem Wasser-Tetrahydrofuran-Gemisch (2/3 v/v) in einer Ausbeute von 76 % der Theorie. Farblose Kristalle.
IR (KBr): 3473.2, 3345.9 ($NH_2$); 1714.4 (C=O) $cm^{-1}$.

Beispiel A3

trans-2-(4-Amino-3,5-dibrombenzoyl)-cyclopropancarbonsäuremethylester

Zu der Lösung von 3.9 g (0.02074 Mol) Trimethyl-oxosulfoniumiodid in 50 ml wasserfreiem Dimethylsulfoxid gab man bei Zimmertemperatur und unter Rühren in kleinen Portionen 0.45 g (0.01781 Mol) 95proz. Natriumhydrid, rührte weitere 30 Minuten bei Zimmertemperatur und tropfte dann die Lösung von 5.8 g (0.01598 Mol) trans-4-(4-Amino-3,5-dibromphenyl)-4-oxo-butensäuremethylester in 50 ml Dimethylsulfoxid ohne äußere Erwärmung zu, wobei die Temperatur der Mischung bis auf 35°C anstieg, und rührte eine weitere Stunde bei Zimmertemperatur. Die Mischung wurde in 500 ml gesättigte wässerige Kochsalz-Lösung eingerührt, danach mit Essigsäureethylester erschöpfend extrahiert. Die vereinigten Essigesterextrakte wurden über Natriumsulfat getrocknet und im Vakuum eingedampft. Der Rückstand ergab nach säulenchromatographischer Reinigung an Kieselgel (30 bis 60 μm) unter Verwendung von EE/Cyclohexan (1/1 v/v) zum Eluieren 2.6 g (43 % der Theorie) eines farblosen Öls.

| | |
|---|---|
| IR (KBr) | 3475, 3363 ($NH_2$); 1728, 1662 (C=O) $cm^{-1}$ |
| MS | $M^+$ = 375/377/379 ($Br_2$) |

Beispiel A4

trans-4-(4-Amino-3,5-dibromphenyl)-4-oxo-butensäuremethylester

Die Mischung aus 5.6 g (0.016 Mol) trans-4-(4-Amino-3,5-dibromphenyl)-4-oxo-butensäure, 50 ml wasserfreiem Methanol und 4.0 g (0.0368 Mol) Trimethylchlorsilan wurde 3 Tage bei Zimmertemperatur gerührt. Das Lösemittel wurde im Vakuum entfernt, der Rückstand zwischen Essigsäureethylester und 10proz. Natriumhydrogencarbonat-Lösung verteilt. Die organische Phase wurde über Natriumsulfat getrocknet, abermals im Vakuum eingedampft und ergab 5.8 g (100 % der Theorie) eines farblosen Öls, das ohne weitere Reinigung verwendet wurde.
MS: $M^+$ = 361/363/365 ($Br_2$)

Beispiel A5

3,4-Dihydro-6-[2-(dimethylamino)ethoxy]-3-(4-piperidinyl)-2(1H)-chinazolinon

Die Mischung aus 0.9 g (0.0022 Mol) 3,4-Dihydro-6-[2-(dimethylamino)ethoxy]-3-(1-phenylmethyl-4-piperidinyl)-2(1H)-chinazolinon, 10 ml Methanol und 0.5 g Palladium(II)-hydroxid (Pearlman's catalyst) wurde bis zur Beendigung der Wasserstoffaufnahme hydriert. Der Katalysator wurde abfiltriert, das Filtrat im Vakuum eingedampft und der verbliebene Rückstand ohne wietere Reinigung in der folgenden Stufe verwendet.
Ausbeute: 0.6 g (86 % der Theorie).

| | |
|---|---|
| IR (KBr) | 1662 (C=O) $cm^{-1}$ |
| MS | $M^+$ = 318 |

Entsprechend wurden erhalten:

| N | B | C | Anmerkungen | % Ausb. | FM | $R_f$ | IR [$cm^{-1}$] |
|---|---|---|---|---|---|---|---|
| N8 | H | - | aus N8-$CH_2$Ph, $H_2$, 10proz. Pd-C, MeOH | 92 | D | 0.23 | 1665 (C=O) |
| N18 | H | - | aus N18-$CH_2$Ph, $H_2$, $Pd(OH)_2$, MeOH | 81 | D | 0.26 | |
| N19 | H | - | aus N19-$CH_2$Ph, $H_2$, $Pd(OH)_2$, MeOH | 85 | | | |
| N21 | H | - | aus N21-$CH_2$Ph, $H_2$, $Pd(OH)_2$, MeOH | 100 | D | 0.27 | |
| N23 | H | - | aus N23-$CH_2$Ph, $H_2$, $Pd(OH)_2$, MeOH | 74 | D | 0.35 | |

Beispiel A6

3,4-Dihydro-6-(4-methyl-1-piperazinyl)-3-(4-piperidinyl)-2(1H)-chinazolinon

Zu der eisgekühlten Lösung von 1.1 g (2.561 mMol) 3,4-Dihydro-3-[1-(1,1-dimethylethoxycarbonyl)-4-piperidinyl]-6-(4-methyl-1-piperazinyl)-2(1H)-chinazolinon in 20 ml Methylenchlorid wurden 2 ml Trifluoressigsäure gegeben. Das Reaktionsgemisch wurde 15 Stunden bei Raumtemperatur und 5 Stunden bei 40°C gerührt und anschließend im Vakuum eingeengt. Der verbleibende Rückstand wurde in 5 ml Wasser aufgenommen, die entstandene Lösung mit Kaliumcarbonat gesättigt und mit Dichlormethan erschöpfend extrahiert. Die vereinigten Extrakte wurden im Vakuum eingeengt. Der erhaltene Rückstand wurde an Kieselgel unter Verwendung von anfangs Dichlormethan/Methanol 9/1 (v/v), dann von Dichlormethan/Methanol/konz. Ammoniak 70/30/3 (v/v/v) zum Eluieren säulenchromatographisch gereinigt. Die geeigneten Fraktionen wurden im Vakuum eingedampft, der verbleibende Rückstand (0.5 g; 59% der Theorie) ohne weitere Reinigung in der folgenden Stufe verwendet.

Beispiel A7

3,4-Dihydro-6-[(1,3-dioxolan-2-yl)methoxy]-3-(1-phenylmethyl-4-piperidinyl)-2(1H)-chinazolinon

Zu der Lösung von 5.0 g (14.82 mMol) 3,4-Dihydro-6-hydroxy-3-(1-phenylmethyl-4-piperidinyl)-2(1H)-chinazolinon in 120 ml wasserfreiem Dimethylformamid gab man portionsweise und unter Rühren bei Zimmertemperatur 0.36 g (14.25 mMol) 95proz. Natriumhydrid und hielt die Mischung anschließend 15 Minuten bei 50°C. Dabei bildete sich ein dicker, farbloser Brei. Nach Zugabe von 5.0 g (37.04 mMol) 2-(Brommethyl)-1,3-dioxolan erhitzte man 90 Minuten auf 90°C. Die erkaltete Mischung wurde in gesättigte wässerige Kochsalz-Lösung eingerührt und mit Essigsäureethylester erschöpfend extrahiert. Die vereinigten Extrakte wurden über Natriumsulfat getrocknet und im Vakuum eingedampft, der verbleibende Rückstand an Kieselgel (30-60 μm) unter Verwendung von Dichlormethan/EE/Cyclohexan/Methanol/konz. Ammoniak 60/16/5/5/0.6 v/v/v/v/v zum Eluieren säulenchromatographisch gereinigt. Aufarbeitung der entsprechenden Fraktionen ergab 2.5 g (41 % der Theorie) eines farblosen Öls vom $R_f$ = 0.47 (Dichlormethan/EE/Cyclohexan/Methanol/konz. Ammoniak 60/16/5/5/0.6 v/v/v/v/v).
IR (KBr): 1662 (C=O) $cm^{-1}$

Entsprechend wurden erhalten:

| N | B | C | Anmerkungen | % Ausb. | FM | $R_f$ |
|---|---|---|---|---|---|---|
| N19 | - | $CH_2Ph$ | aus N7-$CH_2Ph$, $BrCH_2CO_2CH_3$ und NaH in DMF | 63 | | |
| N23 | - | $CH_2Ph$ | aus N33-$CH_2Ph$, $BrCH_2CO_2CH_3$ und NaH in DMF | 17 | D | 0.74 |

Beispiel A8

3,4-Dihydro-6-[2-(dimethylamino)ethoxy]-3-(1-phenylmethyl-4-piperidinyl)-2(1H)-chinazolinon

Das Gemisch aus 1.1 g (3.26 mMol) 3,4-Dihydro-6-hydroxy-3-(1-phenylmethyl-4-piperidinyl)-2(1H)-chinazolinon, 50 ml Tetrahydrofuran, 0.30 g (3.366 mMol) 2-Dimethylaminoethanol, 0.94 g (3.584 mMol) Triphenylphosphin und 0.56 g (3.216 mMol) Azodicarbonsäureester wurde eine Stunde bei Zimmertemperatur, 6 Stunden bei Rückflußtemperatur und abermals 13 Stunden bei Zimmertemperatur gerührt. Das Lösemittel wurde im Vakuum entfernt, der Rückstand an Kieselgel (30-60 μm) unter Verwendung von Dichlormethan/EE/Cyclohexan/Methanol/konz. Ammoniak 60/16/5/5/0.6 v/v/v/v/v zum Eluieren säulenchromatographisch gereinigt. Aufarbeitung der entsprechenden Fraktionen ergab 0.9 g (69 % der Theorie) einer farblosen kristallinen Substanz vom $R_f$ = 0.47 (Dichlormethan/EE/Cyclohexan/Methanol/konz. Ammoniak 60/16/5/5/0.6 v/v/v/v/v).
MS: ESI: $(M+H)^+$ = 409; $(M+2H)^{++}$ = 205; $(M+Na)^+$ = 431

Entsprechend wurden erhalten:

| N | B | C | Anmerkungen | % Ausb. | FM | $R_f$ | MS | IR [$cm^{-1}$] |
|---|---|---|---|---|---|---|---|---|
| N18 | - | $CH_2Ph$ | aus N7-$CH_2Ph$, $(H_3C)_2NCH_2CH_2CH_2OH$, $PPh_3$ und $(NCO_2Et)_2$ in THF | 59 | P | 0.12 | $M^+ = 422$ | 3357, 3271 (NH); 1622 (C=O, C=C) |
| N21 | - | $CH_2Ph$ | aus N7-$CH_2Ph$, $O[(H_2C)_2]_2NCH_2CH_2OH$, $PPh_3$ und $(NCO_2Et)_2$ in THF | 45 | | | | |

Beispiel A9

3,4-Dihydro-3-[1-(1,1-dimethylethoxycarbonyl)-4-piperidinyl]-6-(4-methyl-1-piperazinyl)-2(1H)-chinazolinon

Das Gemisch aus 10.0 g (24.372 mMol) 6-Brom-3,4-dihydro-3-[1-(1,1-dimethylethoxycarbonyl)-4-piperidinyl]-2(1H)-chinazolinon, 2.5 g (24.96 mol) 1-Methylpiperazin, 4.81 g (50.05 mMol) Natrium-tert.-butylat, 285 mg (0.4766 mMol) Bis-(dibenzylidenaceton)-palladium, 305 mg (1.002 mMol) Tris-(o-tolyl)-phosphin und 100 ml Toluol wurde 14 Stunden lang unter Rückfluß gekocht. Nach Zugabe der gleichen Mengen 1-Methylpiperazin, Natrium-tert.-butylat, Bis-(dibenzylidenaceton)-palladium und Tris-(o-tolyl)-phosphin wurde weitere 48 Stunden unter Rückfluß gekocht. Das Gemisch wurde über Aktivkohle filtriert und das Filtrat im Vakuum eingeengt. Der Rückstand wurde zwischen Dichlormethan und Wasser verteilt. Die organische Phase wurde zweimal mit verdünnter wässeriger Zitronensäure-Lösung extrahiert. Die so erhaltenen sauren Auszüge wurden natronalkalisch gestellt und mit Dichlormethan erschöpfend extrahiert. Die vereinigten Dichlormethan-Auszüge wurden im Vakuum eingedampft, der Rückstand an Kieselgel (30-60 µm) unter Verwendung von anfangs Dichlormethan, dann Methanol/konz. Ammoniak 9/1 v/v zum Eluieren säulenchromatographisch gereinigt. Nach üblicher Aufarbeitung der geeigneten Eluate erhielt man 1.1 g (11 % der Theorie) einer farblosen Substanz.

| | |
|---|---|
| IR (KBr) | 1670 (C=O) $cm^{-1}$ |
| MS | $M^+ = 429$ |

Beispiel A10

3,4-Dihydro-7-hydroxy-3-(1-phenylmethyl-4-piperidinyl)-2(1H)-chinazolinon

Die Mischung aus 18.0 g (0.0512 Mol) 3,4-Dihydro-7-methoxy-3-(1-phenylmethyl-4-piperidinyl)-2(1H)-chinazolinon und 100 g Pyridin-hydrochlorid wurde unter Rühren 3 Stunden auf 160°C erhitzt. Nach dem Erkalten löste man das Produkt in 500 ml Wasser, behandelte die erhaltene Lösung vorsichtig mit überschüssigem, festem Natriumhydrogencarbonat, wobei sich ein hochviskoses Öl abschied. Dieses Öl wurde in 150 ml Methanol aufgenommen, die entstandene methanolische Lösung über Aktivkohle geklärt, anschließend wieder im Vakuum vom Lösemittel befreit. Der Rückstand wurde mit 50 ml Acetonitril verrührt und anschließend aufgekocht. Man ließ erkalten, nutschte den entstandenen Niederschlag ab und trocknete ihn im Vakuum und bei Zimmertemperatur.
Ausbeute: 10.8 g (63 % der Theorie).
$R_f = 0.32$ (FM F).

| | |
|---|---|
| IR (KBr) | 1649 (C=O) $cm^{-1}$ |
| MS | $M^+ = 337$ |

Beispiel A11

3,4-Dihydro-7-methoxy-3-(1-phenylmethyl-4-piperidinyl)-2(1H)-chinazolinon

Die Mischung aus 2.5 g (7.682 mMol) 2-Amino-4-methoxy-N-(1-phenylmethyl-4-piperidinyl)-benzylamin, 1.62 g (10 mMol) N,N'-Carbonyldiimidazol und 25 ml Dimethylformamid wurde unter Rühren 2.5 Stunden lang auf 90°C erhitzt. Nach dem Erkalten wurde die Mischung in 100 ml Eiswasser eingerührt, die entstandene Suspension mit 10

ml tert.-Butylmethylether überschichtet, der ausgefallene Niederschlag abgenutscht, mit Wasser und dann mit tert.-Butylmethylether gewaschen. Nach dem Trocknen im Vakuum erhielt man 1.9 g (70 % der Theorie) an farblosen Kristallen.

| | |
|---|---|
| IR (KBr) | 1664 (C=O) $cm^{-1}$ |
| MS | $M^+$ = 351 |

Beispiel A12

2-Amino-4-methoxy-N-(1-phenylmethyl-4-piperidinyl)-benzylamin

Die Lösung von 3.2 g (9.003 mMol) 4-Methoxy-2-nitro-N-(1-phenylmethyl-4-piperidinyl)-benzylamin in 30 ml Methanol wurde in Gegenwart von 1 g 10proz. Rhodiumkohle 5 Stunden lang bei Zimmertemperatur hydriert. Der Katalysator wurde abfiltriert, das Filtrat im Vakuum eingedampft. Man erhielt 2.5 g (85 % der Theorie) eines farblosen hochviskosen Öls, das ohne weitere Reinigung weiterverarbeitet wurde.
$R_f$ = 0.34 (FM F).

| | |
|---|---|
| IR (KBr) | kein C=O |
| MS | $M^+$ = 325 |

Beispiel A12

4-Methoxy-2-nitro-N-(1-phenylmethyl-4-piperidinyl)-benzylamin

Die Mischung aus 3.0 g (16.561 mMol) 4-Methoxy-2-nitrobenzaldehyd, 3.2 g (16.817 mMol) 1-Phenylmethyl-4-piperidinamin und 30 ml Methanol wurde 2 Stunden bei Zimmertemperatur gerührt. Dann gab man 681 mg (18.0 mMol) Natriumborhydrid zu und rührte abermals eine Stunde bei Zimmertemperatur. Die Mischung wurde in 500 ml Eiswasser eingerührt und mit 10proz. Salzsäure vorsichtig angesäuert. Die erhaltene Lösung wurde zweimal mit je 50 ml tert.-Butylmethylether gewaschen, anschließend mit 20proz. Natronlauge alkalisch gestellt und mit tert.-Butylmethylether erschöpfend extrahiert. Die zuletzt erhaltenen Extrakte wurden vereinigt, zweimal mit je 20 ml Wasser gewaschen, über Magnesiumsulfat getrocknet und im Vakuum eingedampft. Das verbleibende farblose Öl wurde ohne weitere Reinigung in der nächsten Stufe verwendet.
Ausbeute: 3.2 g (54 % der Theorie).

| | |
|---|---|
| IR (KBr): | kein C=O |
| MS : | $M^+$ = 355 |

**B. Herstellung der Endverbindungen**

Beispiel 1

cis-3-{1-[2-(4-Chlorbenzoyl)-cyclopropancarbonyl]-4-piperidinyl}-3,4-dihydro-2(1H)-chinazolinon (Lfd. Nr. 1)

Die Mischung aus 1.0 g (4.452 mmol) trans-2-(4-Chlorbenzoyl)-cyclopropancarbonsäure, 0.97 g (4.194 mmol) 3,4-Dihydro-3-(4-piperidinyl)-2(1H)-chinazolinon, 1.4 g (4.36 mmol) TBTU, 0.455 mg (4.5 mmol) Triethylamin und 20 ml Dimethylformamid wurde 5 Stunden bei Raumtemperatur gerührt. Das Reaktionsgemisch wurde im Vakuum vom Lösemittel befreit, mit 300 ml Wasser verdünnt und mit Zitronensäure schwach angesäuert. Der entstandene Niederschlag wurde abgenutscht und mit Wasser, dann mit 5 ml Tetrahydrofuran sorgfältig gewaschen, schließlich im Umlufttrockenschrank bei einer Temperatur von 60°C getrocknet. Man erhielt 1.3 g (71 % der Theorie) eines farblosen, kristallinen Produkts vom Fp. 272-273°C und $R_f$ 0.24 (FM A).

| | |
|---|---|
| IR (KBr) | 1674.1 $cm^{-1}$ (C=O) |
| MS | $M^+$ = 437/439 (Cl) |

Analog wurden hergestellt:

| Lfd. Nr. | N | B | C | Anmerkungen | % Ausb. | FM | $R_f$ | MS | IR [$cm^{-1}$] | Fp. [°C] |
|---|---|---|---|---|---|---|---|---|---|---|
| 2 | N2 | B1 | C2 | THF als LM; DIEA als Base | 49 | A | 0.63 | $M^+$ = 616/618/620 ($Br_2$); ESI: $(M+H)^+$ = 617/619/621 ($Br_2$) | 1684 (C=O) | farblose Kristalle |
| 3 | N2 | B1 | C2 | THF als LM; DIEA als Base | 42 | A | 0.78 | $M^+$ = 574/576/578 ($Br_2$) | 1668 (C=O) | farblose Kristalle |
| 4 | N3 | B1 | C2 | DMF als LM; DIEA als Base | 58 | D<br>A | 0.78<br>0.84 | $M^+$ = 652/654/656/658 ($Br_3$) | 1670 (C=O) | |
| 5 | N4 | B1 | C2 | DMF als LM; DIEA als Base | 57 | A | 0.66 | ESI: $(M+H)^+$ = 587/589/591 ($Br_2$); $(M+Na)^+$ = 609/611/613 ($Br_2$) | 1684 (C=O) | |
| 6 | N5 | B1 | C2 | DMF als LM; DIEA als Base | 26 | D<br>A | 0.4<br>0.73 | $M^+$ = 654/656/658 ($Br_2$) | 3465, 3383 (NH, $NH_2$); 1685 (C=O); 1205, 1165, 1124 ($CF_3$) | |
| 7 | N6 | B1 | C2 | DMF als LM; DIEA als Base | 31 | D<br>A | 0.4<br>0.61 | $M^+$ = 602/604/606 ($Br_2$) | 1676 (C=O) | farblose Kristalle |
| 8 | N7 | B1 | C2 | DMF als LM; DIEA als Base | 13 | D<br>A | 0.7<br>0.73 | $M^+$ = 590/592/594 ($Br_2$) | 3379 (OH, NH); 1709, 1653 (C=O) | |
| 9 | N8 | B1 | C2 | DMF als LM; DIEA als Base | 62 | D<br>A | 0.5<br>0.74 | $M^+$ = 676/678/680 ($Br_2$) | 3460, 3332 (NH, $NH_2$); 1666 (C=O) | |
| 10 | N9 | B1 | C2 | THF als LM; DIEA als Base | 52 | D<br>A | 0.6<br>0.75 | $M^+$ = 654/656/658/660 ($Br_2Cl_2$) | 3462, 3383 (NH, $NH_2$); 1685 (C=O) | |
| 11 | N10 | B1 | C2 | THF als LM; DIEA als Base | 65 | A | 0.36 | $M^+$ = 575/577/579 ($Br_2$) | 3444 (NH, $NH_2$); 1676 (C=O) | |
| 12 | N11 | B1 | C2 | DMF als LM; DIEA als Base | 68 | D<br>A | 0.75<br>0.69 | ESI: $(M+H)^+$ = 617/619/621 ($Br_2$); $(M+Na)^+$ = 639/641/643 ($Br_2$); $(M+K)^+$ = 655/657/659 ($Br_2$) | 1682 (C=O) | |
| 13 | N12 | B1 | C2 | DMF als LM; DIEA als Base | 76 | D<br>A | 0.7<br>0.75 | $M^+$ = 620/622/624 ($Br_2$, Cl) | 1684 (C=O) | |

(fortgesetzt)

| Lfd. Nr. | N | B | C | Anmerkungen | % Ausb. | FM | $R_f$ | MS | IR [$cm^{-1}$] | Fp. [°C] |
|---|---|---|---|---|---|---|---|---|---|---|
| 14 | N13 | B1 | C2 | DMF als LM; DIEA als Base | 63 | D<br>A | 0.65<br>0.73 | $M^+$ = 631/633/635 ($Br_2$) | 3458, 3379 (NH, $NH_2$); 1684 (C=O) | |
| 15 | N14 | B1 | C2 | THF/DMF 1/1 v/v als LM; $NEt_3$ als Base | 62 | G<br>A | 0.39<br>0.52 | $M^+$ = 575/577/579 ($Br_2$) | 1668 (C=O) | farblose Kristalle |
| 16 | N15 | B1 | C2 | DMF als LM; DIEA als Base | 24 | C | 0.20 | $M^+$ = 661/663/665 ($Br_2$) | 1666 (C=O) | 235 (AcOEt) |
| 17 | N16 | B1 | C2 | THF/DMF 1/1 v/v als LM; $NEt_3$ als Base | 11 | C | 0.23 | $M^+$ = 672/674/676 ($Br_2$) | 1666 (C=O) | |
| 18 | N17 | B1 | C2 | DMF als LM; DIEA als Base | 11 | A | 0.71 | $M^+$ = 654/656/658 ($Br_2$) | 3460, 3383 (NH, $NH_2$); 1687 (C=O) | 248 (AcOEt) |
| 19 | N18 | B1 | C2 | THF/DMF 10/1 v/v als LM; $NEt_3$ als Base | 21 | C | 0.11 | $M^+$ = 675/677/679 ($Br_2$) | 1665 (C=O) | |
| 20 | N1 | B1 | C3 | THF als LM; DIEA als Base | 48 | A | 0.58 | | 1657 (C=O) | farblose Kristalle |
| 21 | N4 | B1 | C3 | THF als LM; DIEA als Base | 78 | | | $M^+$ = 587/589/591 ($Br_2$) | 1676 (C=O) | farblose Kristalle |
| 22 | N19 | B1 | C2 | THF/DMF 10/1 v/v als LM; $NEt_3$ als Base | 50 | A | 0.69 | $M^+$ = 662/664/666 ($Br_2$) | 1739, 1666 (C=O) | farblose Kristalle |
| 24 | N21 | B1 | C2 | THF als LM; $NEt_3$ als Base | 50 | A<br>C | 0.15<br>0.85 | $M^+$ = 703/705/707 ($Br_2$) | 1664 (C=O) | |
| 25 | N22 | B1 | C2 | DMF als LM; $NEt_3$ als Base | 76 | A | 0.74 | $M^+$ = 604/606/608 ($Br_2$) | 1666 (C=O) | farblose Kristalle |
| 26 | N23 | B1 | C2 | THF als LM; $NEt_3$ als Base | 24 | A | 0.73 | $M^+$ = 662/664/666 ($Br_2$) | 3379 (NH, $NH_2$); 1755, 1668 (C=O) | farblose Kristalle |
| 28 | N25 | B1 | C2 | THF/DMF 10/1 viv als LM; $NEt_3$ als Base | 91 | A<br>D | 0.78<br>0.75 | $M^+$ = 632/634/636 ($Br_2$) | 3454, 3379 (NH, $NH_2$); 1720, 1670 (C=O) | farblose Kristalle |
| 29 | N26 | B1 | C2 | DMF als LM; $NEt_3$ als Base | 20 | A<br>D | 0.60<br>0.75 | $M^+$ = 611/613/615 ($Br_2$) | 1730 (C=O) | farblose Kristalle |

(fortgesetzt)

| Lfd. Nr. | N | B | C | Anmerkungen | % Ausb. | FM | $R_f$ | MS | IR [$cm^{-1}$] | Fp. [°C] |
|---|---|---|---|---|---|---|---|---|---|---|
| 30 | N27 | B1 | C2 | THF als LM; DIEA als Base; aus $MeO_2CCH_2NH_2 \cdot HCl$ und N24-B1-C2 | 64 | A | 0.53 | $M^+$ = 689/691/693 ($Br_2$) | 1751, 1666 (C=O) | |
| 31 | N28 | B1 | C2 | THF als LM; DIEA als Base; aus $Me_2NCH2CH_2NH\ CH_3$ und N24-B1-C2 | 49 | C | 0.08 | $M^+$ = 702/704/706 ($Br_2$) | 1666 (C=O) | |
| 32 | N29 | B1 | C2 | THF als LM; DIEA als Base; aus $Et_2NH$ und N24-B1-C2 | 20 | A<br>D | 0.49<br>0.55 | $M^+$ = 673/675/677 ($Br_2$) | 1664 (C=O) | |
| 33 | N30 | B1 | C2 | THF als LM; DIEA als Base; aus $(NH_4)_2CO_3$ und N24-B1-C2 | 40 | A<br>D | 0.31<br>0.48 | $M^+$ = 617/619/621 ($Br_2$) | 3363 (NH, $NH_2$); 1666 (C=O) | farblose Kristalle |
| 35 | N32 | B1 | C2 | THF als LM; DIEA als Base | 57 | A | 0.70 | $M^+$ = 587/589/591 ($Br_2$); $(M-H)^-$ = 586/588/590 ($Br_2$), $(M+Na)^+$ = 610/612/614 ($Br_2$) | 3437, 3321 ($NH_2$, NH); 1684 (C=O) | |

Beispiel 2

trans-3-{1-[2-(4-Amino-3,5-dibrombenzoyl)-cyclopropancarbonyl]-4-piperidinyl}-3,4-dihydro-6-(hydroxycarbonylmethoxy)-2(1H)-chinazolinon (Lfd. Nr. 23)

Zu der Lösung von 0.6 g (0.903 mMol) trans-3-{1-[2-(4-Amino-3,5-dibrombenzoyl)-cyclopropancarbonyl]-4-piperidinyl}-3,4-dihydro-6-(methoxycarbonylmethoxy)-2(1H)-chinazolinon (Lfd. Nr. 22) in einem Gemisch aus 10 ml THF und 10 ml Methanol gab man die Lösung von 0.15 g (3.57 mMol) Lithiumhydroxid-hydrat in 10 ml Wasser. Nach 14stündigem Rühren bei Zimmertemperatur wurden die organischen Lösemittel im Vakuum abdestilliert, der verbleibende Rückstand mit 3.6 ml 1N Salzsäure versetzt. Der entstandene Niederschlag wurde abgenutscht und bei 30°C im Vakuum getrocknet. Der Rückstand wurde in Tetrahydrofuran aufgenommen, die entstandene Lösung heiß filtriert und nach dem Erkalten mit Diisopropylether bis zur Beendigung der Fällungsreaktion versetzt. Der Niederschlag wurde abgenutscht. Nach dem Trocknen im Umlufttrockenschrank erhielt man 0.25 g (43 % der Theorie) an farblosen Kristallen. $R_f$ 0.63 (FM C).

| | |
|---|---|
| IR (KBr) | 1730 $cm^{-1}$ (C=O) |
| MS | ESI: $(M-H+2Na)^+$ = 693/695/697 ($Br_2$);<br>$(M-H)^-$ = 647/649/651 ($Br_2$);<br>$(M+Na)^+$ = 671/673/675 ($Br_2$) |

Analog wurden hergestellt:

| Lfd. Nr. | N | B | C | Anmerkungen | % Ausb. | FM | $R_f$ | MS | IR [$cm^{-1}$] | Fp. [°C] |
|---|---|---|---|---|---|---|---|---|---|---|
| 27 | N24 | B1 | C2 | Verseifung des Methylesters Lfd. Nr. 28 mit NaOH in Wasser/MeOH 1/1 (v/v) | 48 | C<br>F | 0.51<br>0.5 | $M^+$ = 618/620/622 ($Br_2$); ESI: $(M-H)^-$ = 617/619/621 ($Br_2$) | 3379 (NH, $NH_2$); 1666 (C=O) | farblose Kristalle |
| 34 | N31 | B1 | C2 | Verseifung des Methylesters Lfd. Nr. 30 mit LiOH in Wasser/THF 1/1 (v/v) | 73 | C<br>F | 0.37<br>0.27 | ESI: $(M-H+2Na)^+$ = 720/722/724 ($Br_2$); $(M+Na)^+$ = 698/700/702 ($Br_2$) | 1738, 1660 (C=O) | farblose Kristalle |

Die nachfolgenden Beispiele beschreiben die Herstellung pharmazeutischer Anwendungsformen. die als Wirkstoff eine beliebige Verbindung der allgemeinen Formel I enthalten:

Beispiel I

Kapseln zur Pulverinhalation mit 1 mg Wirkstoff

Zusammensetzung:
1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel II

Inhalationslösung für Respimat® mit 1 mg Wirkstoff

Zusammensetzung:
1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

Herstellungsverfahren:

Der Wirkstoff und Benzalkoniumchlorid werden in Wasser gelöst und in Respimat®-Kartuschen abgefüllt.

Beispiel III

Inhalationslösung für Vernebler mit 1 mg Wirkstoff

Zusammensetzung:
1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:

Wirkstoff. Natriumchlorid und Benzalkoniumchlorid werden in Wasser gelöst.

Beispiel IV

Treibgas-Dosieraerosol mit 1 mg Wirkstoff

Zusammensetzung:
1 Hub enthält:

| | |
|---|---|
| Wirkstoff | 1.0 mg |
| Lecithin | 0.1 % |
| Treibgas ad | 50.0 µl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispiel V

Nasalspray mit 1 mg Wirkstoff

Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel VI

Injektionslösung mit 5 mg Wirksubstanz pro 5 ml

Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

Beispiel VII

Injektionslösung mit 100 mg Wirksubstanz pro 20 ml

Zusammensetzung:

| | |
|---|---|
| Wirksubstanz | 100 mg |
| Monokaliumdihydrogenphosphat = $KH_2PO_4$ | 12 mg |
| Dinatriumhydrogenphosphat = $Na_2HPO_4 \cdot 2H_2O$ | 2 mg |
| Natriumchlorid | 180 mg |
| Human-Serum-Albumin | 50 mg |
| Polysorbat 80 | 20 mg |
| Wasser für Injektionszwecke ad | 20 ml |

Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

Beispiel VIII

Lyophilisat mit 10 mg Wirksubstanz

Zusammensetzung:

| | |
|---|---|
| Wirksubstanz | 10 mg |
| Mannit | 300 mg |
| Human-Serum-Albumin | 20 mg |

Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.
Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (Wfl) auflösen; in Ampullen abfüllen.

Beispiel IX

Tabletten mit 20 mg Wirksubstanz

Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispiel X

Kapseln mit 20 mg Wirksubstanz

Zusammensetzung:

| | |
|---|---|
| Wirksubstanz | 20 mg |
| Maisstärke | 80 mg |
| Kieselsäure. hochdispers | 5 mg |
| Magnesiumstearat | 2.5 mg |

Herstellung:

Wirksubstanz, Maisstärke und Kieselsäure homogen mischen; mit Magnesiumstearat mischen; Mischung auf

einer Kapselfüllmaschine in Hartgelatine-Kapseln Grösse 3 abfüllen.

Beispiel XI

Zäpfchen mit 50 mg Wirksubstanz

Zusammensetzung:

| | |
|---|---|
| Wirksubstanz | 50 mg |
| Hartfett (Adeps solidus) q.s. ad | 1700 mg |

Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

Beispiel XII

Injektionslösung mit 10 mg Wirksubstanz pro 1 ml

Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

Herstellung:

Mannitol in Wasser für Injektionszwecke auflösen (WfI); Human-Serum-Albumin zugeben; Wirkstoff unter Erwärmen auflösen; mit WfI auf Ansatzvolumen auffüllen; unter Stickstoff-Begasung in Ampullen abfüllen.

## Patentansprüche

1. Cyclopropane der allgemeinen Formel

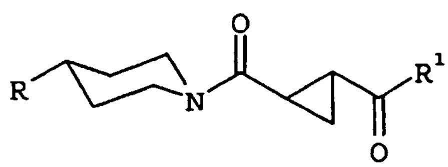

(I),

in der

R einen gesättigten, einfach oder zweifach ungesättigten 5- bis 7-gliedrigen Aza-, Diaza-, Triaza-, Oxaza-, Thiaza-, Thiadiaza- oder S,S-Dioxido-thiadiaza-Heterocyclus,

wobei die vorstehend erwähnten Heterocyclen über ein Kohlenstoff- oder Stickstoffatom verknüpft sein und ein oder zwei Carbonylgruppen benachbart zu einem Stickstoffatom enthalten können,

an einem der Stickstoffatome durch eine Alkylgruppe substituiert sein können,

an einem oder an zwei Kohlenstoffatomen durch eine verzweigte oder unverzweigte Alkylgruppe, durch eine Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolyl-Gruppe substituiert sein können, wobei die Substituenten gleich oder verschieden sein können,

und wobei eine olefinische Doppelbindung eines der vorstehend erwähnten ungesättigten Heterocyclen mit einem Benzol-, Pyridin-, Diazin-, 1,3-Oxazol-, Thiophen-, Furan-, Thiazol-, Pyrrol-, N-Methyl-pyrrol-, Chinolin-, Imidazol- oder N-Methyl-imidazol-Ring kondensiert sein kann,

wobei die in R enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolyl-Gruppen sowie benzo-, thieno-, pyrido- und diazinokondensierten Heterocyclen im Kohlenstoffgerüst zusätzlich durch Fluor-, Chlor- oder Bromatome, durch Alkyl-, Dialkylaminoalkoxy-, Nitro-, Alkylthio-, Alkylsulfinyl-, Alkylsulfonyl-, Alkylsulfonylamino-, Phenyl-, Trifluormethyl-, Alkoxycarbonyl-, Alkoxycarbonylalkyl-, Alkoxycarbonylalkoxy-, Hydroxycarbonylalkoxy-, Carboxy-, Carboxyalkyl-, Dialkylaminoalkyl-, Hydroxy-, Amino-, Acetylamino-, Propionylamino-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, (4-Morpholinyl)carbonyl-, (1-Pyrrolidinyl) carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Aminocarbonylaminoalkyl-, Alkylaminocarbonylamino-, Alkanoyl-, Cyan-, Trifluormethoxy-, Trifluormethylthio-, Trifluormethylsulfinyl-, Trifluormethylsulfonyl- oder Cycloalkylgruppen mit 3 bis 8 Kohlenstoffatomen,

durch 4- bis 8-gliedrige Alkyleniminogruppen, in denen eine Methylengruppe in 3-, 4- oder 5-Position durch ein Sauerstoffatom oder eine Methyliminogruppe ersetzt sein kann,

durch Alkoxygruppen, die in ω-Stellung durch einen 5- bis 7-gliedrigen Heteroalicyclus substituiert sein können, wobei der Heteroalicyclus über ein Kohlenstoff- oder Stickstoffatom verknüpft ist und ein oder zwei nicht direkt aneinander gebundene Heteroatome ausgewählt aus Sauerstoff und Stickstoff enthält,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenyl-, 1-Naphthyl-, 2-Naphthyl-, 1,2,3,4-Tetrahydro-1-naphthyl-, 1H-Indol-3-yl-, 1-Methyl-1H-indol-3-yl-, 1-Formyl-1H-indol-3-yl-, 4-Imidazolyl-, 1-Methyl-4-imidazolyl-, 2-Thienyl-, 3-Thienyl-, Thiazolyl-, 1H-Indazol-3-yl-, 1-Methyl-1H-indazol-3-yl-, Benzo[b]fur-3-yl-, Benzo[b]thien-3-yl-, Pyridinyl-, Chinolinyl- oder Isochinolinylgruppe,

wobei die vorstehend erwähnten aromatischen und heteroaromatischen Reste im Kohlenstoffgerüst zusätzlich durch Fluor-, Chlor- oder Bromatome, durch verzweigte oder unverzweigte Alkylgruppen, durch Cycloalkylgruppen mit 3 bis 8 Kohlenstoffatomen, durch Phenylalkyl-, Alkenyl-, Alkoxy-, Phenyl-, Phenylalkoxy-, Trifluormethyl-, Alkoxycarbonylalkyl-, Carboxyalkyl-, Alkoxycarbonyl-, Carboxy-, Dialkylaminoalkyl-, Dialkylaminoalkoxy-, Nitro-, Hydroxy-, Amino-, Acetylamino-, Propionylamino-, Methylsulfonyloxy-, Aminocarbonyl-, Alkylaminocarbonyl-, Dialkylaminocarbonyl-, Alkanoyl-, Cyan-, Tetrazolyl-, Phenyl-, Pyridinyl-, Thiazolyl-, Furyl-, Trifluormethoxy-, Trifluormethylthio-, Trifluormethylsulfinyl- oder Trifluormethylsulfonylgruppen mono-, di- oder trisubstituiert sein können und die Substituenten gleich oder verschieden sein können,

wobei die in den vorstehend genannten Gruppen enthaltenen Hydroxy-, Amino-, Indolyl- und Imidazolylgruppen mit den aus der Peptidchemie geläufigen Schutzresten substituiert sein können, und

alle vorstehend genannten Alkyl- und Alkoxygruppen sowie die innerhalb der anderen genannten Reste vorhandenen Alkyl- oder Alkylenteile, sofern nichts anderes angegeben ist, 1 bis 5 Kohlenstoffatome umfassen können,

wobei unter in der Peptidchemie geläufigen Schutzgruppen

eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,

eine Alkoxycarbonylgruppe mit insgesamt 1 bis 5 Kohlenstoffatomen im Alkylteil,

die Allyloxycarbonyl-, 2,2,2-Trichlor-(1,1-dimethylethoxy)carbonyl- oder 9-Fluorenylmethoxycarbonyl-Gruppe oder

die Formyl-, Acetyl- oder Trifluoracetylgruppe zu verstehen ist,

bedeuten, deren Tautomere, deren Diastereomere, deren Enantiomere, deren Gemische und deren Salze.

**2.** Verbindungen der allgemeinen Formel I gemäß Anspruch 1, in der,

R einen einfach oder zweifach ungesättigten 5- bis 7-gliedrigen Aza-, Diaza-, Triaza- oder Thiaza-Heterocyclus,

wobei die vorstehend erwähnten Heterocyclen über ein Kohlenstoff- oder Stickstoffatom verknüpft sein und eine oder zwei Carbonylgruppen benachbart zu einem Stickstoffatom enthalten können,

an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppe substituiert sein können,

und wobei-eine olefinische Doppelbindung eines der vorstehend erwähnten ungesättigten Heterocyclen mit einem Benzol-, Pyridin-, Diazin- oder Chinolin-Ring kondensiert sein kann,

wobei die in R enthaltenen Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppen sowie benzo-, pyrido- und diazinokondensierten Heterocyclen im Kohlenstoffgerüst zusätzlich durch Fluor-, Chlor- oder Bromatome, durch Alkyl-, Dialkylaminoalkoxy-, Nitro-, Trifluormethyl-, Alkoxycarbonyl-, Alkoxycarbonylalkyl-, Alkoxycarbonylalkoxy-, Hydroxycarbonylalkoxy-, Carboxy-, Carboxyalkyl-, Dialkylaminoalkyl-, Hydroxy-, Amino-, Acetylamino-, Propionylamino-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, Aminocarbonylamino-, Aminocarbonylaminoalkyl-, Alkylaminocarbonylamino-, Alkanoyl- oder Trifluormethoxy-Gruppen,

durch 5- bis 7-gliedrige Alkyleniminogruppen, in denen eine Methylengruppe in 3-, 4- oder 5-Position durch ein Sauerstoffatom oder eine Methyliminogruppe ersetzt sein kann,

durch Alkoxygruppen, die in ω-Stellung durch einen 5- bis 7-gliedrigen Heteroalicyclus substituiert sein können, wobei der Heteroalicyclus über ein Kohlenstoff- oder Stickstoffatom verknüpft ist und ein oder zwei nicht direkt aneinander gebundene Heteroatome ausgewählt aus Sauerstoff und Stickstoff enthält,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenyl-, 1-Naphthyl- oder 2-Naphthylgruppe,

wobei die vorstehend erwähnten aromatischen Reste durch Fluor-, Chlor- oder Bromatome, durch verzweigte oder unverzweigte Alkylgruppen, Alkoxy-, Trifluormethyl-, Nitro-, Hydroxy-, Amino- oder Acetylaminogruppen mono-, di- oder trisubstituiert und die Substituenten gleich oder verschieden sein können,

und wobei alle vorstehend genannten Alkyl- und Alkoxygruppen sowie die innerhalb der anderen genannten Reste vorhandenen Alkyl- oder Alkylenteile, sofern nichts anderes angegeben ist, 1 bis 4 Kohlenstoffatome umfassen können,

bedeuten, deren Tautomere, deren Diastereomere, deren Enantiomere und deren Salze.

**3.** Verbindungen der allgemeinen Formel I gemäß Anspruch 1, in der,

R einen einfach ungesättigten 5- bis 7-gliedrigen Diaza- oder Triaza-Heterocyclus,

wobei die vorstehend erwähnten Heterocyclen über ein Stickstoffatom verknüpft sind,

eine Carbonylgruppe benachbart zu einem Stickstoffatom enthalten und

an einem Kohlenstoffatom durch eine Phenyl-Gruppe substituiert oder

eine olefinische Doppelbindung eines der vorstehend erwähnten ungesättigten Heterocyclen mit einem Benzol-, Pyridin- oder Chinolin-Ring kondensiert sein kann,

und wobei die in R enthaltenen Phenyl-Gruppen sowie benzo- und pyrido-kondensierten Heterocyclen im Kohlenstoffgerüst zusätzlich durch Fluor-, Chlor- oder Bromatome, durch Alkyl-, Dialkylaminoalkoxy-, Nitro-, Trifluormethyl-, Alkoxycarbonyl-, Alkoxycarbonylalkoxy-, Hydroxycarbonylalkoxy-, Carboxy-, Hydroxy-, Aminocarbonyl-, Alkylaminocarbonyl-, Dialkylaminocarbonyl-, [N-Alkyl-N-(dialkylaminoalkyl)amino]carbonyl-, [(Hydroxycarbonylalkyl)amino]carbonyl-, [(Alkoxycarbonylalkyl)amino]carbonyl-, Alkanoyl- oder Trifluormethoxy-Gruppen, durch 5- bis 7-gliedrige Alkyleniminogruppen, in denen eine Methylengruppe in 3- oder 4-Position durch ein Sauerstoffatom oder eine Methyliminogruppe ersetzt sein kann, beispielsweise 1-Pyrrolidinyl-, 1-Piperidinyl-, 4-Methyl-1-piperazinyl-, 4-Methyl-1,4-diazacyclohept-1-yl- oder 4-Morpholinylgruppen,

durch Alkoxygruppen, die in ω-Stellung durch einen 5- oder 6-gliedrigen Heteroalicyclus substituiert sein können, wobei der Heteroalicyclus über ein Kohlenstoffatom verknüpft ist und in 2- und 2'-Stellung je ein Sauerstoffatom enthält oder über ein Kohlenstoff- oder Stickstoffatom verknüpft ist und ein oder zwei nicht direkt aneinander gebundene Stickstoffatome oder ein Sauerstoff- und ein Stickstoffatom, die durch mindestens eine Methylengruppe voneinander getrennt sind, enthält, beispielsweise Methoxy-, Ethoxy-, Propoxy-, 2,5-Dioxacyclopentylmethoxy-, 2,6-Dioxacyclohexylmethoxy-, 2-(1-Pyrrolidinyl)ethoxy-, 2-(1-Piperidinyl)ethoxy-, 2-(4-Methyl-1-piperazinyl)ethoxy- oder 2-(4-Morpholinyl)ethoxy-Gruppen,

mono-, di- oder trisubstituiert sein können, wobei eine Mehrfachsubstitution durch cyclische Reste oder solche Reste, die einen Carbo- oder Heterocyclus enthalten, ausgeschlossen ist und wobei die Substituenten gleich oder verschieden sein können,

und $R^1$ eine Phenylgruppe,

die durch Fluor-, Chlor- oder Bromatome, durch Alkoxy-, Trifluormethyl-, Nitro-, Hydroxy- oder Aminogruppen mono-, di- oder trisubstituiert sein kann, wobei die Substituenten gleich oder verschieden sein können,

wobei alle vorstehend genannten Alkyl- und Alkoxygruppen sowie die innerhalb der anderen genannten Reste vorhandenen Alkyl- oder Alkylenteile, sofern nichts anderes angegeben ist, 1 bis 3 Kohlenstoffatome umfassen können,

bedeuten, deren Tautomere, deren Diastereomere, deren Enantiomere und deren Salze.

**4.** Verbindungen der allgemeinen Formel I gemäß Anspruch 1, in der,

R eine 3,4-Dihydro-2(1H)-oxochinazolin-3-yl-, 1,3-Dihydro-4-phenyl-2H-2-oxoimidazol-1-yl-, 2,4-Dihydro-5-phenyl-3(3H)-oxo-1,2,4-triazol-2-yl-, 3,4-Dihydro-2(1H)-oxopyrido[4,3-d]pyrimidin-3-yl-, 3,4-Dihydro-2(1H)-oxopyrido[3,4-d]pyrimidin-3-yl- oder 1,3-Dihydro-2(2H)-oxoimidazo[4,5-c]chinolin-3-yl-Gruppe,

wobei die vorstehend erwähnten mono- und bicyclischen Heterocyclen im Kohlenstoffgerüst durch Fluor-,

Chlor- oder Bromatome mono- oder disubstituiert oder durch eine 4-Methyl-1-piperazinyl-, 2,5-Dioxacyclopentylmethoxy-, Methoxy-, 2-(4-Morpholinyl)ethoxy-, 2-Dimethylaminoethoxy-, 3-Dimethylaminopropoxy-, Methoxycarbonylmethoxy-, Hydroxycarbonylmethoxy-, Nitro-, Trifluormethyl-, Methoxycarbonyl-, Carboxy-, Hydroxy-, Aminocarbonyl-, Diethylaminocarbonyl-, [N-(2-Dimethylaminoethyl)-N-methylamino]carbonyl-, [(Methoxycarbonylmethyl)amino]carbonyl- oder [(Hydroxycarbonylmethyl)amino]carbonyl-Gruppe monosubstituiert sein können,
und $R^1$ eine Phenylgruppe,

die durch Fluor-, Chlor- oder Bromatome, durch Hydroxy- oder Aminogruppen mono-, di- oder trisubstituiert sein kann, wobei die Substituenten gleich oder verschieden sein können, beispielsweise die 4-Chlorphenyl-, 4-Amino-3,5-dibromphenyl- oder 3,5-Dibrom-4-Hydroxyphenylgruppe,

bedeuten, deren Tautomere, deren Diastereomere, deren Enantiomere und deren Salze.

**5.** Folgende Verbindungen der allgemeinen Formel I gemäß Anspruch 1:

(1) cis-3-{1-[2-(4-Chlorbenzoyl)-cyclopropancarbonyl]-4-piperidinyl}-3,4-dihydro-2(1H)-chinazolinon

(2)trans-1-{1-[2-(4-Amino-3,5-dibrombenzoyl)-cyclopropancarbonyl]-4-piperidinyl}-1,3-dihydro-4-(3-methoxyphenyl)-2(2H)-imidazolon

(3) trans-3-{1-[2-(4-Amino-3,5-dibrombenzoyl)-cyclopropancarbonyl]-4-piperidinyl}-3,4-dihydro-2(1H)-chinazolinon

(4) trans-3-{1-[2-(4-Amino-3,5-dibrombenzoyl)-cyclopropancarbonyl]-4-piperidinyl}-6-brom-3,4-dihydro-2(1H)-chinazolinon

(5) trans-1-{1-[2-(4-Amino-3,5-dibrombenzoyl)-cyclopropancarbonyl]-4-piperidinyl}-1,3-dihydro-4-phenyl-2(2H)-imidazolon

(6) trans-1-{1-[2-(4-Amino-3,5-dibrombenzoyl)-cyclopropancarbonyl]-4-piperidinyl}-1,3-dihydro-4-[3-(trifluormethyl)phenyl]-2(2H)-imidazolon

(7) trans-1-{1-[2-(4-Amino-3,5-dibrombenzoyl)-cyclopropancarbonyl]-4-piperidinyl}-1,3-dihydro-4-(3-hydroxyphenyl)-2(2H)-imidazolon

(8) trans-3-{1-[2-(4-Amino-3,5-dibrombenzoyl)-cyclopropancarbonyl]-4-piperidinyl}-3,4-dihydro-6-hydroxy-2(1H)-chinazolinon

(9) trans-3-{1-[2-(4-Amino-3,5-dibrombenzoyl)-cyclopropancarbonyl]-4-piperidinyl}-3,4-dihydro-6-[(1,3-dioxolan-2-yl)methoxy]-2(1H)-chinazolinon

(10) trans-1-{1-[2-(4-Amino-3,5-dibrombenzoyl)-cyclopropancarbonyl]-4-piperidinyl}-4-(3,4-dichlorphenyl)-1,3-dihydro-2(2H)-imidazolon

(11) trans-3-{1-[2-(4-Amino-3,5-dibrombenzoyl)-cyclopropancarbonyl]-4-piperidinyl}-3,4-dihydro-2(1H)-pyrido[4,3-d]pyrimidinon

(12) trans-1-{1-[2-(4-Amino-3,5-dibrombenzoyl)-cyclopropancarbonyl]-4-piperidinyl}-1,3-dihydro-4-(2-methoxyphenyl)-2(2H)-imidazolon

(13) trans-1-{1-[2-(4-Amino-3,5-dibrombenzoyl)-cyclopropancarbonyl]-4-piperidinyl}-4-(3-chlorphenyl)-1,3-dihydro-2(2H)-imidazolon

(14) trans-1-{1-[2-(4-Amino-3,5-dibrombenzoyl)-cyclopropancarbonyl]-4-piperidinyl}-1,3-dihydro-4-(3-nitrophenyl)-2(2H)-imidazolon

(15) trans-3-{1-[2-(4-Amino-3,5-dibrombenzoyl)-cyclopropancarbonyl]-4-piperidinyl}-3,4-dihydro-2(1H)-pyrido[3,4-d]pyrimidinon

(16) trans-3-{1-[2-(4-Amino-3,5-dibrombenzoyl)-cyclopropancarbonyl]-4-piperidinyl}-3,4-dihydro-6-[2-(dimethylamino)ethoxy]-2(1H)-chinazolinon

(17) trans-3-{1-[2-(4-Amino-3,5-dibrombenzoyl)-cyclopropancarbonyl]-4-piperidinyl}-3,4-dihydro-6-(4-methyl-1-piperazinyl)-2(1H)-chinazolinon

(18)trans-1-{1-[2-(4-Amino-3,5-dibrombenzoyl)-cyclopropancarbonyl]-4-piperidinyl}-1,3-dihydro-4-[2-(trifluormethyl)phenyl]-2(2H)-imidazolon

(19) trans-3-{1-[2-(4-Amino-3,5-dibrombenzoyl)-cyclopropancarbonyl]-4-piperidinyl}-3,4-dihydro-6-[3-(dimethylamino)propoxy]-2(1H)-chinazolinon

(20) trans-3-{1-[2-(3,5-Dibrom-4-hydroxybenzoyl)-cyclopropancarbonyl]-4-piperidinyl}-3,4-dihydro-2(1H)-chinazolinon

(21) trans-1-{1-[2-(3,5-Dibrom-4-hydroxybenzoyl)-cyclopropancarbonyl]-4-piperidinyl}-1,3-dihydro-4-phenyl-2(2H)-imidazolon

(22) trans-3-{1-[2-(4-Amino-3,5-dibrombenzoyl)-cyclopropancarbonyl]-4-piperidinyl}-3,4-dihydro-6-(methoxycarbonylmethoxy)-2(1H)-chinazolinon.

(23) trans-3-{1-[2-(4-Amino-3,5-dibrombenzoyl)-cyclopropancarbonyl]-4-piperidinyl}-3,4-dihydro-6-(hydroxycarbonylmethoxy)-2(1H)-chinazolinon

(24) trans-3-{1-[2-(4-Amino-3,5-dibrombenzoyl)-cyclopropancarbonyl]-4-piperidinyl}-3,4-dihydro-6-[2-(4-morpholinyl)ethoxy]-2(1H)-chinazolinon

(25) trans-3-{1-[2-(4-Amino-3,5-dibrombenzoyl)-cyclopropancarbonyl]-4-piperidinyl}-3,4-dihydro-7-methoxy-2(1H)-chinazolinon

(26) trans-3-{1-[2-(4-Amino-3,5-dibrombenzoyl)-cyclopropancarbonyl]-4-piperidinyl}-3,4-dihydro-7-(methoxycarbonylmethoxy)-2(1H)-chinazolinon

(27) trans-3-{1-[2-(4-Amino-3,5-dibrombenzoyl)-cyclopropancarbonyl]-4-piperidinyl}-7-carboxy-3,4-dihydro-2(1H)-chinazolinon

(28) trans-3-{1-[2-(4-Amino-3,5-dibrombenzoyl)-cyclopropancarbonyl]-4-piperidinyl}-7-methoxycarbonyl-3,4-dihydro-2(1H)-chinazolinon

(29) trans-3-{1-[2-(4-Amino-3,5-dibrombenzoyl)-cyclopropancarbonyl]-4-piperidinyl}-1,3-dihydro-2(2H)-imidazo[4,5-c]chinolinon

(30) trans-3-{1-[2-(4-Amino-3,5-dibrombenzoyl)-cyclopropancarbonyl]-4-piperidinyl}-3,4-dihydro-7-{[(methoxycarbonylmethyl)-amino]carbonyl}-2(1H)-chinazolinon

(31) trans-3-{1-[2-(4-Amino-3,5-dibrombenzoyl)-cyclopropancarbonyl]-4-piperidinyl}-3,4-dihydro-7-{[N-(2-dimethylaminoethyl)-N-methylamino]carbonyl}-2(1H)-chinazolinon

(32) trans-3-{1-[2-(4-Amino-3,5-dibrombenzoyl)-cyclopropancarbonyl]-4-piperidinyl}-7-diethylaminocarbonyl-3,4-dihydro-2(1H)-chinazolinon

(33) trans-7-Aminocarbonyl-3-{1-[2-(4-amino-3,5-dibrombenzoyl)-cyclopropancarbonyl]-4-piperidinyl}-3,4-dihydro-2(1H)-chinazolinon

(34) trans-3-{1-[2-(4-Amino-3,5-dibrombenzoyl)-cyclopropancarbonyl]-4-piperidinyl}-3,4-dihydro-7-{[(hydroxycarbonylmethyl)amino]carbonyl}-2(1H)-chinazolinon

(35) trans-1-{1-[2-(4-Amino-3,5-dibrombenzoyl)-cyclopropancarbonyl]-4-piperidinyl}-2,4-dihydro-5-phenyl-3(3H)-1,2,4-triazolon,

sowie deren Salze.

**6.** Folgende Verbindungen der allgemeinen Formel I gemäß Anspruch 1:

(a) trans-1-{1-[2-(4-Amino-3,5-dibrombenzoyl)-cyclopropancarbonyl]-4-piperidinyl}-1,3-dihydro-4-(3-methoxyphenyl)-2(2H)-imidazolon,

(b) trans-3-{1-[2-(4-Amino-3,5-dibrombenzoyl)-cyclopropancarbonyl]-4-piperidinyl}-3,4-dihydro-2(1H)-chinazolinon,

(c) trans-1-{1-[2-(4-Amino-3,5-dibrombenzoyl)-cyclopropancarbonyl]-4-piperidinyl}-1,3-dihydro-4-phenyl-2(2H)-imidazolon,

(d) trans-1-{1-[2-(4-Amino-3,5-dibrombenzoyl)-cyclopropancarbonyl]-4-piperidinyl}-1,3-dihydro-4-(3-hydroxyphenyl)-2(2H)-imidazolon,

(e) trans-3-{1-[2-(4-Amino-3,5-dibrombenzoyl)-cyclopropancarbonyl]-4-piperidinyl}-3,4-dihydro-6-hydroxy-2(1H)-chinazolinon,

(f) trans-3-{1-[2-(4-Amino-3,5-dibrombenzoyl)-cyclopropancarbonyl]-4-piperidinyl}-3,4-dihydro-6-[(1,3-dioxolan-2-yl)methoxy]-2(1H)-chinazolinon,

(g) trans-1-{1-[2-(4-Amino-3,5-dibrombenzoyl)-cyclopropancarbonyl]-4-piperidinyl}-4-(3-chlorphenyl)-1,3-dihydro-2(2H)-imidazolon,

(h) trans-3-{1-[2-(4-Amino-3,5-dibrombenzoyl)-cyclopropancarbonyl]-4-piperidinyl}-3,4-dihydro-6-[3-(dimethylamino)propoxy]-2(1H)-chinazolinon,

(i) trans-3-{1-[2-(4-Amino-3,5-dibrombenzoyl)-cyclopropancarbonyl]-4-piperidinyl}-3,4-dihydro-6-(methoxycarbonylmethoxy)-2(1H)-chinazolinon,

(j) trans-3-{1-[2-(4-Amino-3,5-dibrombenzoyl)-cyclopropancarbonyl]-4-piperidinyl}-3,4-dihydro-6-(hydroxycarbonylmethoxy)-2(1H)-chinazolinon und

(k) trans-1-{1-[2-(4-Amino-3,5-dibrombenzoyl)-cyclopropancarbonyl]-4-piperidinyl}-2,4-dihydro-5-phenyl-3(3H)-1,2,4-triazolon

sowie deren Salze.

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das CGRP-antagonistische Eigenschaften aufweist.

**10.** Verwendung einer Verbindung nach mindestens einem der Ansprüche 1 bis 7 zur Herstellung eines Arzneimittels, das zur akuten und prophylaktischen Behandlung von Kopfschmerzen, zur Behandlung des nicht-insulinabhängigen Diabetes mellitus, von cardiovaskulären Erkrankungen, Erkrankungen der Haut, von entzündlichen Erkrankungen, der allergischen Rhinitis, von Asthma, von Erkrankungen, die mit einer überschießenden Gefäßerweiterung und **dadurch** bedingter verringerter Gewebedurchblutung einhergehen, der Morphintoleranz oder zur Bekämpfung menopausaler Hitzewallungen geeignet ist.

**11.** Verfahren zur Herstellung eines Arzneimittels gemäß Anspruch 8, **dadurch gekennzeichnet, daß** auf nichtchemischem Wege eine Verbindung nach mindestens einem der Ansprüche 1 bis 7 in einen oder mehrere inerte

Trägerstoffe und/oder Verdünnungsmittel eingearbeitet wird.

**12.** Verfahren zur Herstellung der Verbindungen der allgemeinen Formel I gemäß den Ansprüchen 1 bis 7, **dadurch gekennzeichnet, daß**

a) eine Carbonsäure der allgemeinen Formel

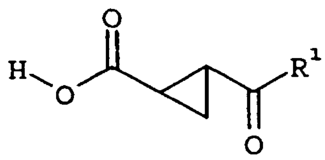
(II),

in der
$R^1$ wie in den Ansprüchen 1 bis 6 erwähnt definiert ist,
mit einer Verbindung der allgemeinen Formel

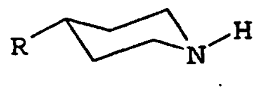
(III),

in der
R wie in den Ansprüchen 1 bis 6 erwähnt definiert ist, gekuppelt wird oder

b) eine Verbindung der allgemeinen Formel

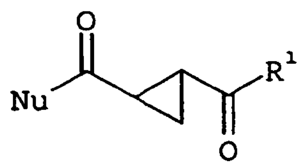
(IV),

in der
$R^1$ wie in den Ansprüchen 1 bis 6 erwähnt definiert ist und
Nu eine Austrittsgruppe bedeutet,
mit einer Verbindung der allgemeinen Formel

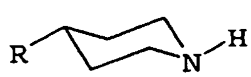
(III),

in der
R wie in den Ansprüchen 1 bis 6 erwähnt definiert ist, gekuppelt wird oder

c) eine Verbindung der allgemeinen Formel

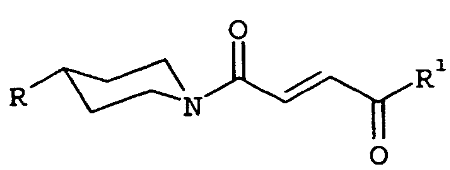
(V),

in der
R und $R^1$ wie in den Ansprüchen 1 bis 6 erwähnt definiert sind, cyclopropanyliert wird oder

d) zur Herstellung einer Verbindung der allgemeinen Formel I, in der mindestens einer der Reste R und $R^1$ eine oder mehrere Carboxygruppen enthält,
ein Carbonsäureester der allgemeinen Formel

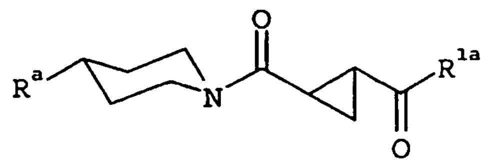 (Ia),

in der
$R^a$ und $R^{1a}$ mit der Maßgabe, daß mindestens einer dieser Reste eine oder mehrere Alkoxycarbonylgruppen enthält, die die in den Ansprüchen 1 bis 6 für R bzw. $R^1$ angegebenen Bedeutungen besitzen, alkalisch verseift wird
und gewünschtenfalls anschließend eine so erhaltenes Salz durch Behandlung mit einer verdünnten organischen oder anorganischen Säure in die zugrundeliegende Säure übergeführt wird oder

e) zur Herstellung eine Verbindung der allgemeinen Formel I, in der
der Rest R im Kohlenstoffgerüst durch eine Aminocarbonyl-, Alkylaminocarbonyl-, Dialkylaminocarbonyl-, [N-Alkyl-N-(dialkylaminoalkyl)amino]carbonyl-, Hydroxycarbonylalkylaminocarbonyl-, Alkoxycarbonylalkylaminocarbonyl-, (4-Morpholinyl)carbonyl-, (1-Pyrrolidinyl)carbonyl-, (1-Piperidinyl)carbonyl-, (Hexahydro-1-azepinyl)carbonyl- oder (4-Methyl-1-piperazinyl)carbonylGruppe gleichartig mono-, di- oder trisubstituiert ist, eine Verbindung der allgemeinen Formel

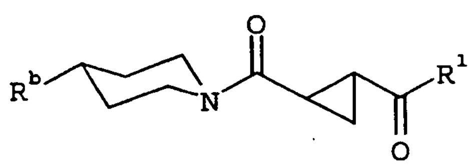 (Ib),

in der
$R^1$ wie in den Ansprüchen 1 bis 6 erwähnt definiert ist und
der Rest $R^b$ mit der Maßgabe, daß er im Kohlenstoffgerüst durch die Carboxy-Gruppe mono-, di- oder trisubstituiert ist, die in den Ansprüchen 1 bis 6 für R angegebenen Bedeutungen hat,
mit Ammoniak, mit einem entsprechenden Alkylamin, N-Alkyl-N-(dialkylaminoalkyl)amin, Hydroxycarbonylalkylamin, Alkoxycarbonylalkylamin oder Dialkylamin gekuppelt wird und
gewünschtenfalls anschließend eine so erhaltene Verbindung der allgemeinen Formel I in ihre Diastereomeren und/oder Enantiomeren aufgetrennt wird und/oder
eine so erhaltene Verbindung der allgemeinen Formel I in ihre Salze, insbesondere in ihre physiologisch verträglichen Salze übergeführt wird.

## Claims

1. Cyclopropanes of general formula

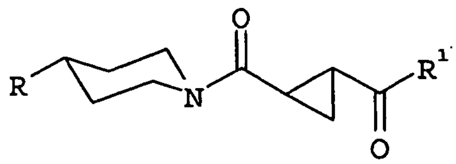

(I),

wherein
R denotes a saturated, mono- or di-unsaturated 5- to 7-membered aza, diaza, triaza, oxaza, thiaza, thiadiaza or S,S-dioxido-thiadiaza heterocyclic group,
whilst the abovementioned heterocyclic groups are linked via a carbon or nitrogen atom and
may contain one or two carbonyl groups adjacent to a nitrogen atom,
may be substituted by an alkyl group at one of the nitrogen atoms,
may be substituted at one or two carbon atoms by a straight-chain or branched alkyl group, by a phenyl, phenylmethyl, naphthyl, biphenylyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl group, whilst the substituents may be identical or different,
and wherein an olefinic double bond of one of the abovementioned unsaturated heterocyclic groups may be fused with a benzene, pyridine, diazine, 1,3-oxazole, thiophene, furan, thiazole, pyrrole, N-methylpyrrole, quinoline, imidazole or N-methyl-imidazole ring,
while the phenyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl groups contained in R and the benzo-, thieno-, pyrido- and diazino-fused heterocyclic groups in the carbon skeleton may additionally be mono-, di- or trisubstituted by fluorine, chlorine or bromine atoms, by alkyl, dialkylaminoalkoxy, nitro, alkylthio, alkylsulphinyl, alkylsulphonyl, alkylsulphonylamino, phenyl, trifluoromethyl, alkoxycarbonyl, alkoxycarbonylalkyl, alkoxycarbonylalkoxy, hydroxycarbonylalkoxy, carboxy, carboxyalkyl, dialkylaminoalkyl, hydroxy, amino, acetylamino, propionylamino, aminocarbonyl, alkylaminocarbonyl, dialkylaminocarbonyl, [N-alkyl-N-(dialkylaminoalkyl)amino]carbonyl, [(hydroxycarbonylalkyl)amino]carbonyl, [(alkoxycarbonylalkyl)amino]carbonyl, (4-morpholinyl)carbonyl, (1-pyrrolidinyl)carbonyl, (1-piperidinyl)carbonyl, (hexahydro-1-azepinyl)carbonyl, (4-methyl-1-piperazinyl)carbonyl, methylenedioxy, aminocarbonylamino, aminocarbonylaminoalkyl, alkylaminocarbonylamino, alkanoyl, cyano, trifluoromethoxy, trifluoromethylthio, trifluoromethylsulphinyl, trifluoromethylsulphonyl or cycloalkyl groups with 3 to 8 carbon atoms,
by 4- to 8-membered alkyleneimino groups wherein a methylene group in the 3-, 4- or 5-position may be replaced by an oxygen atom or a methylimino group,
by alkoxy groups which may be substituted in the ω position by a 5- to 7-membered heteroalicyclic group, where the heteroalicyclic group is linked via a carbon or nitrogen atom and contains one or two heteroatoms not directly connected to each other selected from among oxygen and nitrogen,
while multiple substitution by cyclic groups or those groups which contain a carbocyclic or heterocyclic group is excluded and wherein the substituents may be identical or different,
and $R^1$ denotes a phenyl, 1-naphthyl, 2-naphthyl, 1,2,3,4-tetrahydro-1-naphthyl, 1H-indol-3-yl, 1-methyl-1H-indol-3-yl, 1-formyl-1H-indol-3-yl, 4-imidazolyl, 1-methyl-4-imidazolyl, 2-thienyl, 3-thienyl, thiazolyl, 1H-indazol-3-yl, 1-methyl-1H-indazol-3-yl, benzo[b]fur-3-yl, benzo[b]thien-3-yl, pyridinyl, quinolinyl or isoquinolinyl group,
whilst the abovementioned aromatic and heteroaromatic groups may additionally be mono-, di- or trisubstituted in the carbon skeleton by fluorine, chlorine or bromine atoms, by branched or unbranched alkyl groups, by cycloalkyl groups with 3 to 8 carbon atoms, by phenylalkyl, alkenyl, alkoxy, phenyl, phenylalkoxy, trifluoromethyl, alkoxycarbonylalkyl, carboxyalkyl, alkoxycarbonyl, carboxy, dialkylaminoalkyl, dialkylaminoalkoxy, nitro, hydroxy, amino, acetylamino, propionylamino, methylsulphonyloxy, aminocarbonyl, alkylaminocarbonyl, dialkylaminocarbonyl, alkanoyl, cyano, tetrazolyl, phenyl, pyridinyl, thiazolyl, furyl, trifluoromethoxy, trifluoromethylthio, trifluoromethylsulphinyl or trifluoromethylsulphonyl groups and the substituents may be identical or different,
while the hydroxy, amino, indolyl and imidazolyl groups contained in the abovementioned groups may be substituted by protecting groups familiar from peptide chemistry, and
all the abovementioned alkyl and alkoxy groups and the alkyl or alkylene moieties present within the other groups specified may contain 1 to 5 carbon atoms, unless otherwise stated,
while by protecting groups familiar from peptide chemistry are meant
a phenylalkoxycarbonyl group with 1 to 3 carbon atoms in the alkoxy moiety, optionally substituted in the phenyl nucleus by a halogen atom, by a nitro or phenyl group or by one or two methoxy groups,
an alkoxycarbonyl group having a total of 1 to 5 carbon atoms in the alkyl moiety,
the allyloxycarbonyl, 2,2,2-trichloro-(1,1-dimethylethoxy)carbonyl or 9-fluorenylmethoxycarbonyl group or

the formyl, acetyl or trifluoroacetyl group,
the tautomers, diastereomers, enantiomers, mixtures thereof and the salts thereof.

**2.** Compounds of general formula I according to claim 1, wherein
R denotes a mono- or di-unsaturated 5- to 7-membered aza, diaza, triaza or thiaza heterocyclic group,
whilst the abovementioned heterocyclic groups are linked via a carbon or nitrogen atom and
may contain one or two carbonyl groups adjacent to a nitrogen atom,
may be substituted at a carbon atom by a phenyl, pyridinyl, diazinyl, thienyl, pyrrolyl, 1,3-thiazolyl, isoxazolyl, pyrazolyl or 1-methylpyrazolyl group,
and wherein an olefinic double bond of one of the abovementioned unsaturated heterocyclic groups may be fused to a benzene, pyridine, diazine or quinoline ring,
while the phenyl, pyridinyl, diazinyl, thienyl, pyrrolyl, 1,3-thiazolyl, isoxazolyl, pyrazolyl or 1-methylpyrazolyl groups contained in R and the benzo-, pyrido- and diazino-fused heterocyclic groups in the carbon skeleton may additionally be mono-, di- or trisubstituted by fluorine, chlorine or bromine atoms, by alkyl, dialkylaminoalkoxy, nitro, trifluoromethyl, alkoxycarbonyl, alkoxycarbonylalkyl, alkoxycarbonylalkoxy, hydroxycarbonylalkoxy, carboxy, carboxyalkyl, dialkylaminoalkyl, hydroxy, amino, acetylamino, propionylamino, aminocarbonyl, alkylaminocarbonyl, dialkylaminocarbonyl, [N-alkyl-N-(dialkylaminoalkyl)amino] carbonyl, [(hydroxycarbonylalkyl)amino]carbonyl, [(alkoxycarbonylalkyl)amino]carbonyl, aminocarbonylamino, aminocarbonylaminoalkyl, alkylaminocarbonylamino, alkanoyl or trifluoromethoxy groups,
by 5- to 7-membered alkyleneimino groups wherein a methylene group in the 3-, 4- or 5-position may be replaced by an oxygen atom or a methylimino group,
by alkoxy groups which may be substituted in the ω-position by a 5- to 7-membered heteroalicyclic group, where the heteroalicyclic group is linked via a carbon or nitrogen atom and contains one or two heteroatoms not directly connected to each other selected from among oxygen and nitrogen,
while multiple substitution by cyclic groups or those groups which contain a carbocyclic or heterocyclic group is excluded and wherein the substituents may be identical or different,
and $R^1$ denotes a phenyl, 1-naphthyl or 2-naphthyl group,
while the abovementioned aromatic groups may be mono-, di- or trisubstituted by fluorine, chlorine or bromine atoms, by branched or unbranched alkyl groups, alkoxy, trifluoromethyl, nitro, hydroxy, amino or acetylamino groups and the substituents may be identical or different,
and wherein all the abovementioned alkyl and alkoxy groups and the alkyl or alkylene moieties present within the other groups mentioned may contain 1 to 4 carbon atoms, unless otherwise stated,
the tautomers, diastereomers, enantiomers and salts thereof.

**3.** Compounds of general formula I according to claim 1, wherein
R denotes a monounsaturated 5- to 7-membered diaza or triaza heterocyclic group,
while the abovementioned heterocyclic groups are linked via a nitrogen atom,
may contain a carbonyl group adjacent to a nitrogen atom and
may be substituted at a carbon atom by a phenyl group or
an olefinic double bond of one of the abovementioned unsaturated heterocyclic groups may be fused with a benzene, pyridine or quinoline ring,
and the phenyl groups contained in R as well as the benzo- and pyrido-fused heterocyclic groups in the carbon skeleton may additionally be mono-, di- or trisubstituted by fluorine, chlorine or bromine atoms, by alkyl, dialkylaminoalkoxy, nitro, trifluoromethyl, alkoxycarbonyl, alkoxycarbonylalkoxy, hydroxycarbonylalkoxy, carboxy, hydroxy, aminocarbonyl, alkylaminocarbonyl, dialkylaminocarbonyl, [N-alkyl-N-(dialkylaminoalkyl)-amino]carbonyl, [(hydroxycarbonylalkyl)amino]carbonyl,
[(alkoxycarbonylalkyl)amino]carbonyl, alkanoyl or trifluoromethoxy groups,
by 5- to 7-membered alkyleneimino groups wherein a methylene group in the 3- or 4-position may be replaced by an oxygen atom or a methylimino group, for example 1-pyrrolidinyl, 1-piperidinyl, 4-methyl-1-piperazinyl, 4-methyl-1,4-diazacyclohept-1-yl or 4-morpholinyl groups,
by alkoxy groups which may be substituted in the ω-position by a 5- or 6-membered heteroalicyclic group, wherein the heteroalicyclic group is linked via a carbon atom and contains an oxygen atom in each of the 2-and 2'-positions or is linked via a carbon or nitrogen atom and contains one or two nitrogen atoms not directly linked to one another or an oxygen and a nitrogen atom which are separated from each other by at least one methylene group, for example methoxy, ethoxy, propoxy, 2,5-dioxacyclopentylmethoxy, 2,6-dioxacyclohexylmethoxy, 2-(1-pyrrolidinyl)ethoxy, 2-(1-piperidinyl)ethoxy, 2-(4-methyl-1-piperazinyl) ethoxy or 2-(4-morpholinyl)ethoxy groups,
while multiple substitution by cyclic groups or those groups which contain a carbocyclic or heterocyclic group is excluded and wherein the substituents may be identical or different,

and $R^1$ denotes a phenyl group
which may be mono-, di- or trisubstituted by fluorine, chlorine or bromine atoms, by alkoxy, trifluoromethyl, nitro, hydroxy or amino groups, while the substituents may be identical or different,
and wherein all the abovementioned alkyl and alkoxy groups and the alkyl or alkylene moieties present within the other groups mentioned may contain 1 to 3 carbon atoms, unless otherwise stated,
the tautomers, diastereomers, enantiomers and salts thereof.

**4.** Compounds of general formula I according to claim 1, wherein
R denotes a 3,4-dihydro-2(1H)-oxoquinazolin-3-yl, 1,3-dihydro-4-phenyl-2H-2-oxoimidazol-1-yl, 2,4-dihydro-5-phenyl-3(3H)-oxo-1,2,4-triazol-2-yl, 3,4-dihydro-2(1H)-oxopyrido[4,3-d]pyrimidin-3-yl, 3,4-dihydro-2(1H)-oxopyrido[3,4-d]pyrimidin-3-yl or 1,3-dihydro-2(2H)-oxoimidazo[4,5-c]quinolin-3-yl group,
wherein the abovementioned mono- and bicyclic heterocyclic groups may be mono- or disubstituted in the carbon skeleton by fluorine, chlorine or bromine atoms or may be monosubstituted by a 4-methyl-1-piperazinyl, 2,5-dioxacyclopentylmethoxy, methoxy, 2-(4-morpholinyl)ethoxy, 2-dimethylaminoethoxy, 3-dimethylaminopropoxy, methoxycarbonylmethoxy, hydroxycarbonylmethoxy, nitro, trifluoromethyl, methoxycarbonyl, carboxy, hydroxy, aminocarbonyl, diethylaminocarbonyl, [N-(2-dimethylaminoethyl)-N-methylamino]carbonyl, [(methoxycarbonylmethyl)amino]carbonyl or [(hydroxycarbonylmethyl)amino]carbonyl group,
and $R^1$ denotes a phenyl group,
which may be mono-, di- or trisubstituted by fluorine, chlorine or bromine atoms or by hydroxy or amino groups, wherein the substituents may be identical or different, for example the 4-chlorophenyl, 4-amino-3,5-dibromophenyl or 3,5-dibromo-4-hydroxyphenyl group,
the tautomers, diastereomers, enantiomers and salts thereof.

**5.** The following compounds of general formula I according to claim 1:

(1) cis-3-{1-[2-(4-chlorobenzoyl)-cyclopropanecarbonyl]-4-piperidinyl}-3,4-dihydro-2(1H)-quinazolinone

(2) trans-1-{1-[2-(4-amino-3,5-dibromobenzoyl)-cyclopropanecarbonyl]-4-piperidinyl}-1,3-dihydro-4-(3-methoxyphenyl)-2(2H)-imidazolone,

(3) trans-3-{1-[2-(4-amino-3,5-dibromobenzoyl)-cyclopropanecarbonyl]-4-piperidinyl}-3,4-dihydro-2(1H)-quinazolinone,

(4) trans-3-{1-[2-(4-amino-3,5-dibromobenzoyl)-cyclopropanecarbonyl]-4-piperidinyl}-6-bromo-3,4-dihydro-2(1H)-quinazolinone,

(5) trans-1-{1-[2-(4-amino-3,5-dibromobenzoyl)-cyclopropanecarbonyl]-4-piperidinyl}-1,3-dihydro-4-phenyl-2(2H)-imidazolone,

(6) trans-1-{1-[2-(4-amino-3,5-dibromobenzoyl)-cyclopropanecarbonyl]-4-piperidinyl}-1,3-dihydro-4-[3-(trifluoromethyl)phenyl]-2(2H)-imidazolone,

(7) trans-1-{1-[2-(4-amino-3,5-dibromobenzoyl)-cyclopropanecarbonyl]-4-piperidinyl}-1,3-dihydro-4-(3-hydroxyphenyl)-2(2H)-imidazolone,

(8) trans-3-{1-[2-(4-amino-3,5-dibromobenzoyl)-cyclopropanecarbonyl]-4-piperidinyl}-3,4-dihydro-6-hydroxy-2(1H)-quinazolinone,

(9) trans-3-{1-[2-(4-amino-3,5-dibromobenzoyl)-cyclopropanecarbonyl]-4-piperidinyl}-3,4-dihydro-6-[(1,3-dioxolan-2-yl)methoxy]-2(1H)-quinazolinone,

(10) trans-1-{1-[2-(4-amino-3,5-dibromobenzoyl)-cyclopropanecarbonyl]-4-piperidinyl}-4-(3,4-dichlorophenyl)-1,3-dihydro-2(2H)-imidazolone,

(11) trans-3-{1-[2-(4-amino-3,5-dibromobenzoyl)-cyclopropanecarbonyl]-4-piperidinyl}-3,4-dihydro-2(1H)-pyrido[4,3-d]pyrimidinone

(12) trans-1-{1-[2-(4-amino-3,5-dibromobenzoyl)-cyclopropanecarbonyl]-4-piperidinyl}-1,3-dihydro-4-(2-methoxyphenyl)-2(2H)-imidazolone

(13) trans-1-{1-[2-(4-amino-3,5-dibromobenzoyl)-cyclopropanecarbonyl]-4-piperidinyl}-4-(3-chlorophenyl)-1, 3-dihydro-2(2H)-imidazolone

(14) trans-1-{1-[2-(4-amino-3,5-dibromobenzoyl)-cyclopropanecarbonyl]-4-piperidinyl}-1,3-dihydro-4-(3-nitrophenyl)-2(2H)-imidazolone

(15) trans-3-{1-[2-(4-amino-3,5-dibromobenzoyl)-cyclopropanecarbonyl]-4-piperidinyl}-3,4-dihydro-2(1H)-pyrido[3,4-d]pyrimidinone

(16) trans-3-{1-[2-(4-amino-3,5-dibromobenzoyl)-cyclopropanecarbonyl]-4-piperidinyl}-3,4-dihydro-6-[2-(dimethylamino)ethoxy]-2(1H)-quinazolinone

(17) trans-3-{1-[2-(4-amino-3,5-dibromobenzoyl)-cyclopropanecarbonyl]-4-piperidinyl}-3,4-dihydro-6-(4-methyl-1-piperazinyl)-2(1H)-quinazolinone

(18) trans-1-{1-[2-(4-amino-3,5-dibromobenzoyl)-cyclopropanecarbonyl]-4-piperidinyl}-1,3-dihydro-4-[2-(trifluoromethyl)phenyl]-2(2H)-imidazolone

(19) trans-3-{1-[2-(4-amino-3,5-dibromobenzoyl)-cyclopropanecarbonyl]-4-piperidinyl}-3,4-dihydro-6-[3-(dimethylamino)propoxy]-2(1H)-quinazolinone

(20) trans-3-{1-[2-(3,5-dibromo-4-hydroxybenzoyl)-cyclopropanecarbonyl]-4-piperidinyl}-3,4-dihydro-2(1H)-quinazolinone

(21) trans-1-{1-[2-(3,5-dibromo-4-hydroxybenzoyl)-cyclopropane-carbonyl]-4-piperidinyl}-1,3-dihydro-4-phenyl-2(2H)-imidazolone

(22) trans-3-{1-[2-(4-amino-3,5-dibromobenzoyl)-cyclopropanecarbonyl]-4-piperidinyl}-3,4-dihydro-6-(methoxycarbonylmethoxy)-2(1H)-quinazolinone

(23)trans-3-{1-[2-(4-amino-3,5-dibromobenzoyl)-cyclopropanecarbonyl]-4-piperidinyl}-3,4-dihydro-6-(hydroxycarbonylmethoxy)-2(1H)-quinazolinone

(24) trans-3-{1-[2-(4-amino-3,5-dibromobenzoyl)-cyclopropanecarbonyl]-4-piperidinyl}-3,4-dihydro-6-[2-(4-morpholinyl)ethoxy]-2(1H)-quinazolinone

(25) trans-3-{1-[2-(4-amino-3,5-dibromobenzoyl)-cyclopropanecarbonyl]-4-piperidinyl}-3,4-dihydro-7-methoxy-2(1H)-quinazolinone

(26) trans-3-{1-[2-(4-amino-3,5-dibromobenzoyl)-cyclopropanecarbonyl]-4-piperidinyl}-3,4-dihydro-7-(methoxycarbonylmethoxy)-2(1H)-quinazolinone

(27) trans-3-{1-[2-(4-amino-3,5-dibromobenzoyl)-cyclopropanecarbonyl]-4-piperidinyl}-7-carboxy-3,4-dihydro-2(1H)-quinazolinone

(28) trans-3-{1-[2-(4-amino-3,5-dibromobenzoyl)-cyclopropanecarbonyl]-4-piperidinyl}-7-methoxycarbonyl-3, 4-dihydro-2(1H)-quinazolinone

(29) trans-3-{1-[2-(4-amino-3,5-dibromobenzoyl)-cyclopropanecarbonyl]-4-piperidinyl}-1,3-dihydro-2(2H)-imidazo[4,5-c]quinolinone

(30) trans-3-{1-[2-(4-amino-3,5-dibromobenzoyl)cyclopropanecarbonyl]-4-piperidinyl}-3,4-dihydro-7-{[(methoxycarbonylmethyl)-amino]carbonyl}-2(1H)-quinazolinone

(31) trans-3-{1-[2-(4-amino-3,5-dibromobenzoyl)-cyclopropanecarbonyl]-4-piperidinyl}-3,4-dihydro-7-{[N-(2-dimethylaminoethyl)-N-methylamino]carbonyl}-2(1H)-quinazolinone

(32) trans-3-{1-[2-(4-amino-3,5-dibronibenzoyl)-cyclopropanecarbonyl]-4-piperidinyl}-7-diethylaminocarbonyl-3,4-dihydro-2(1H)-quinazolinone

(33) trans-7-aminocarbonyl-3-{1-[2-(4-amino-3,5-dibromobenzoyl)-cyclopropanecarbonyl]-4-piperidinyl}-3,4-dihydro-2(1H)-quinazolinone

(34) trans-3-{1-[2-(4-amino-3,5-dibromobenzoyl)-cyclopropanecarbonyl]-4-piperidinyl}-3,4-dihydro-7-{[(hydroxycarbonylmethyl)-amino]carbonyl}-2(1H)-quinazolinone

(35) trans-1-{1-[2-(4-amino-3,5-dibromobenzoyl)-cyclopropanecarbonyl]-4-piperidinyl}-2,4-dihydro-5-phenyl-3(3H)-1,2,4-triazolone

and the salts thereof.

**6.** The following compounds of general formula I according to claim 1:

(a) trans-1-{1-[2-(4-amino-3,5-dibromobenzoyl)-cyclopropanecarbonyl]-4-piperidinyl}-1,3-dihydro-4-(3-methoxyphenyl)-2(2H)-imidazolone,

(b) trans-3-{1-[2-(4-amino-3,5-dibromobenzoyl)-cyclopropanecarbonyl]-4-piperidinyl}-3,4-dihydro-2(1H)-quinazolinone,

(c) trans-1-{1-[2-(4-amino-3,5-dibromobenzoyl)-cyclopropanecarbonyl]-4-piperidinyl}-1,3-dihydro-4-phenyl-2(2H)-imidazolone,

(d) trans-1-{1-[2-(4-amino-3,5-dibromobenzoyl)-cyclopropanecarbonyl]-4-piperidinyl}-1,3-dihydro-4-(3-hydroxyphenyl)-2(2H)-imidazolone,

(e) trans-3-{1-[2-(4-amino-3,5-dibromobenzoyl)-cyclopropanecarbonyl]-4-piperidinyl}-3,4-dihydro-6-hydroxy-2(1H)-quinazolinone,

(f)trans-3-{1-[2-(4-amino-3,5-dibromobenzoyl)-cyclopropanecarbonyl]-4-piperidinyl}-3,4-dihydro-6-[(1,3-dioxolan-2-yl)methoxy]-2(1H)-quinazolinone,

(g) trans-1-{1-[2-(4-amino-3,5-dibromobenzoyl)-cyclopropanecarbonyl]-4-piperidinyl}-4-(3-chlorophenyl)-1,3-dihydro-2(2H)-imidazolone,

(h) trans-3-{1-[2-(4-amino-3,5-dibromobenzoyl)-cyclopropanecarbonyl]-4-piperidinyl}-3,4-dihydro-6-[3-(dimethylamino)propoxy]-2(1H)-quinazolinone,

(i) trans-3-{1-[2-(4-amino-3,5-dibromobenzoyl)-cyclopropanecarbonyl]-4-piperidinyl}-3,4-dihydro-6-(methoxycarbonylmethoxy)-2(1H)-quinazolinone,

(j) trans-3-{1-[2-(4-amino-3,5-dibromobenzoyl)-cyclopropanecarbonyl]-4-piperidinyl}-3,4-dihydro-6-(hydroxycarbonylmethoxy)-2(1H)-quinazolinone and

(k) trans-1-{1-[2-(4-amino-3,5-dibromobenzoyl)-cyclopropanecarbonyl]-4-piperidinyl}-2,4-dihydro-5-phenyl-3(3H)-1,2,4-triazolone

and the salts thereof.

**7.** Physiologically acceptable salts of the compounds according to at least one of claims 1 to 6 with inorganic or organic acids or bases.

**8.** Pharmaceutical compositions containing a compound according to at least one of claims 1 to 6 or a physiologically acceptable salt according to claim 7 optionally together with one or more inert carriers and/or diluents.

**9.** Use of a compound according to at least one of claims 1 to 7 for preparing a pharmaceutical composition which

has CGRP-antagonistic properties.

**10.** Use of a compound according to at least one of claims 1 to 7 for preparing a pharmaceutical composition which is suitable for the acute and prophylactic treatment of headaches, for treating non-insulin-dependent diabetes mellitus, cardiovascular diseases, skin diseases, inflammatory diseases, allergic rhinitis, asthma, diseases which are accompanied by excessive vasodilatation and consequent reductions in blood flow through the tissues, morphine tolerance or for controlling menopausal hot flushes.

**11.** Process for preparing a pharmaceutical composition according to claim 8, **characterised in that** a compound according to at least one of claims 1 to 7 is incorporated in one or more inert carriers and/or diluents by a non-chemical method.

**12.** Process for preparing the compounds of general formula I according to claims 1 to 7, **characterised in that**

a) a carboxylic acid of general formula

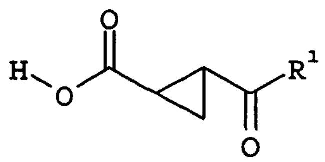

(II),

wherein
$R^1$ is defined as in claims 1 to 6,
is coupled with a compound of general formula

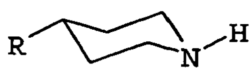

(III),

wherein
R is defined as in claims 1 to 6, or

b) a compound of general formula

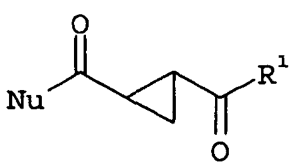

(IV),

wherein
$R^1$ is defined as in claims 1 to 6 and
Nu denotes a leaving group,
is coupled with a compound of general formula

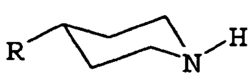

(III),

wherein
$R^1$ is defined as in claims 1 to 6, or

c) a compound of general formula

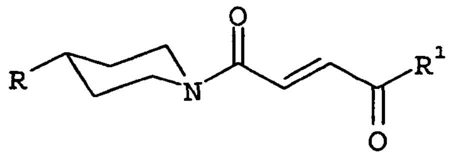

(V),

wherein
R and $R^1$ are defined as in claims 1 to 6 is cyclopropanylated, or

d) in order to prepare a compound of general formula I wherein at least one of the groups R and $R^1$ contains one or more carboxy groups,
a carboxylic acid ester of general formula

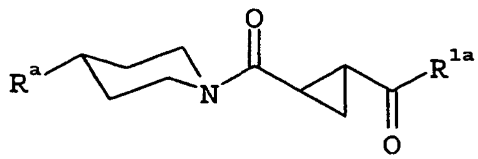

(Ia),

wherein
$R^a$ and $R^{1a}$ have the meanings given in claims 1 to 6 for R and $R^1$, respectively, with the proviso that at least one of these groups contains one or more alkoxycarbonyl groups, is subjected to alkaline saponification and if desired a salt thus obtained is subsequently converted into the basic acid by treating with a dilute organic or inorganic acid, or

e) in order to prepare a compound of general formula I wherein
the group R in the carbon skeleton is similarly mono-, di- or trisubstituted by an aminocarbonyl, alkylaminocarbonyl, dialkylaminocarbonyl, [N-alkyl-N-(dialkylaminoalkyl) amino] carbonyl, hydroxycarbonylalkylaminocarbonyl, alkoxycarbonylalkylaminocarbonyl, (4-morpholinyl)carbonyl, (1-pyrrolidinyl)carbonyl, (1-piperidinyl) carbonyl, (hexahydro-1-azepinyl)carbonyl or (4-methyl-1-piperazinyl)carbonyl group:

a compound of general formula

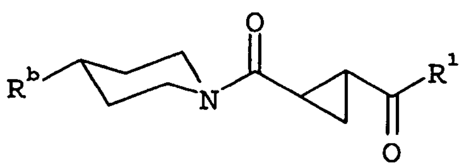

(Ib),

wherein
$R^1$ is defined as in claims 1 to 6 and
the group $R^b$ has the meanings given for R in claims 1 to 6, with the proviso that it is mono-, di- or trisubstituted in the carbon skeleton by the carboxy group,
is coupled with ammonia, with a corresponding alkylamine, N-alkyl-N-(dialkylaminoalkyl) amine, hydroxycarbonylalkylamine, alkoxycarbonylalkylamine or dialkylamine, and
subsequently, if desired, a compound of general formula I thus obtained is resolved into the diastereomers and/or enantiomers thereof and/or
a compound of general formula I thus obtained is converted into the salts thereof, particularly the physiologically acceptable salts thereof.

**Revendications**

**1.** Cyclopropanes de formule générale

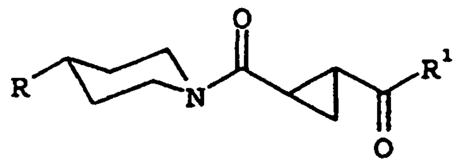

(I),

où
R représente un hétérocycle aza, diaza, triaza, oxaza, thiaza, thiadiaza ou S,S-dioxydo-thiadiaza saturé, une ou deux fois insaturé à 5 à 7 chaînons,
où les hétérocycles cités précédemment sont liés par un atome de carbone ou d'azote et
peuvent contenir un ou deux groupes carbonyle voisins d'un atome d'azote,
peuvent être substitués sur l'un des atomes d'azote par un groupe alkyle,
peuvent être substitués sur un ou sur deux atomes de carbone par un groupe alkyle ramifié ou non ramifié, par un groupe phényle, phénylméthyle, naphtyle, biphénylyle, pyridinyle, diazinyle, furyle, thiényle, pyrrolyle, 1,3-oxazolyle, 1,3-thiazolyle, isoxazolyle, pyrazolyle, 1-méthylpyrazolyle, imidazolyle ou 1-méthylimidazolyle, où les substituants peuvent être identiques ou différents,
et où une double liaison oléfinique de l'un des hétérocycles insaturés cités précédemment peut être condensée avec un cycle benzène, pyridine, diazine, 1,3-oxazole, thiophène, furane, thiazole, pyrrole, N-méthyl-pyrrole, quinoléine, imidazole ou N-méthyl-imidazole,
où les groupes phényle, pyridinyle, diazinyle, furyle, thiényle, pyrrolyle, 1,3-oxazolyle, 1,3-thiazolyle, isoxazolyle, pyrazolyle, 1-méthylpyrazolyle, imidazolyle ou 1-méthylimidazolyle contenus dans R ainsi que les hétérocycles benzo, thiéno, pyrido et diazinocondensés peuvent être mono-, di- ou trisubstitués en outre dans le squelette carboné par des atomes de fluor, de chlore ou de brome, par des groupes alkyle, dialkylaminoalcoxy, nitro, alkylthio, alkylsulfinyle, alkylsulfonyle, alkylsulfonylamino, phényle, trifluorométhyle, alcoxycarbonyle, alcoxycarbonylalkyle, alcoxycarbonylalcoxy, hydroxycarbonylalcoxy, carboxy, carboxyalkyle, dialkylaminoalkyle, hydroxy, amino, acétylamino, propionylamino, aminocarbonyle, alkylaminocarbonyle, dialkylaminocarbonyle, [N-alkyl-N-(dialkylaminoalkyl)amino]carbonyle, [(hydroxycarbonylalkyl)amino]carbonyle, [(alcoxycarbonylalkyl)amino]carbonyle, (4-morpholinyl)carbonyle, (1-pyrrolidinyl)carbonyle, (1-pipéridinyl)carbonyle, (hexahydro-1-azépinyl)carbonyle, (4-méthyl-1-pipérazinyl)carbonyle, méthylènedioxy, aminocarbonylamino, aminocarbonylaminoalkyle, alkylaminocarbonylamino, alcanoyle, cyano, trifluorométhoxy, trifluorométhylthio, trifluorométhylsulfinyle, trifluorométhylsulfonyle ou cycloalkyle à 3 à 8 atomes de carbone,
par des groupes alkylèneimino à 4 à 8 chaînons, où un groupe méthylène en position 3, 4 ou 5 peut être remplacé par un atome d'oxygène ou par un groupe méthylimino,
par des groupes alcoxy qui peuvent être substitués en position ω par un hétéroalicycle à 5 à 7 chaînons, où l'hétéroalicycle est lié par un atome de carbone
ou d'azote et contient un ou deux hétéroatomes non liés directement l'un à l'autre choisis parmi l'oxygène et l'azote,
où une substitution multiple par des groupements cycliques ou par des groupements qui contiennent un carbo- ou hétérocycle est exclue et où les substituants peuvent être identiques ou différents,
et $R^1$ représente un groupe phényle, 1-naphtyle, 2-naphtyle, 1,2,3,4-tétrahydro-1-naphtyle, 1H-indol-3-yle, 1-méthyl-1H-indol-3-yle, 1-formyl-1H-indol-3-yle, 4-imidazolyle, 1-méthyl-4-imidazolyle, 2-thiényle, 3-thiényle, thiazolyle, 1H-indazol-3-yle, 1-méthyl-1H-indazol-3-yle, benzo[b]fur-3-yle, benzo[b]thién-3-yle, pyridinyle, quinolinyle ou isoquinolinyle,
où les groupements aromatiques et hétéroaromatiques cités précédemment peuvent être mono-, di- ou trisubstitués en outre dans le squelette carboné par des atomes de fluor, de chlore ou de brome, par des groupes alkyle ramifiés ou non ramifiés, par des groupes cycloalkyle à 3 à 8 atomes de carbone, par des groupes phénylalkyle, alcényle, alcoxy, phényle, phénylalcoxy, trifluorométhyle, alcoxycarbonylalkyle, carboxyalkyle, alcoxycarbonyle, carboxy, dialkylaminoalkyle, dialkylaminoalcoxy, nitro, hydroxy, amino, acétylamino, propionylamino, méthylsulfonyloxy, aminocarbonyle, alkylaminocarbonyle, dialkylaminocarbonyle, alcanoyle, cyano, tétrazolyle, phényle, pyridinyle, thiazolyle, furyle, trifluorométhoxy, trifluorométhylthio, trifluorométhylsulfinyle ou trifluorométhylsulfonyle et les substituants peuvent être identiques ou différents,
où les groupes hydroxy, amino, indolyle et imidazolyle contenus dans les groupes cités précédemment peuvent être substitués avec les groupements protecteurs courants en chimie des peptides,
et

tous les groupes alkyle et alcoxy cités précédemment ainsi que les parties alkyle ou alkylène présentes dans les autres groupements cités peuvent comprendre, sauf indication contraire, 1 à 5 atomes de carbone,
où, par groupes protecteurs courants en chimie des peptides
on doit comprendre un groupe phénylalcoxycarbonyle à 1 à 3 atomes de carbone dans la partie alcoxy éventuellement substitué dans le noyau phényle par un atome d'halogène, par un groupe nitro ou phényle, par un deux groupes méthoxy,
un groupe alcoxycarbonyle ayant au total 1 à 5 atomes de carbone dans la partie alkyle,
le groupe allyloxycarbonyle, 2,2,2-trichloro-(1,1-diméthyléthoxy)carbonyle ou 9-fluorénylméthoxycarbonyle ou le groupe formyle, acétyle ou trifluoroacétyle,
leurs tautomères, leurs diastéréoisomères, leurs énantiomères, leurs mélanges et leurs sels.

**2.** Composés de formule générale I selon la revendication 1 où
R représente un hétérocycle aza, diaza, triaza ou thiaza une ou deux fois insaturé à 5 à 7 chaînons,
où les hétérocycles cités précédemment sont liés par un atome de carbone ou d'azote et
peuvent contenir un ou deux groupes carbonyle voisins d'un atome d'azote,
peuvent être substitués sur un atome de carbone par un groupe phényle, pyridinyle, diazinyle, thiényle, pyrrolyle, 1,3-thiazolyle, isoxazolyle, pyrazolyle ou 1-méthylpyrazolyle,
et où une double liaison oléfinique de l'un des hétérocycles insaturés cités précédemment peut être condensée avec un cycle benzène, pyridine, diazine ou quinoléine,
où les groupes phényle, pyridinyle, diazinyle, thiényle, pyrrolyle, 1,3-thiazolyle, isoxazolyle, pyrazolyle ou 1-méthylpyrazolyle contenus dans R ainsi que les hétérocycles benzo-, pyrido- et diazinocondensés peuvent être mono-, di- ou trisubstitués en outre dans le squelette carboné par des atomes de fluor, de chlore ou de brome, par des groupes alkyle, dialkylaminoalcoxy, nitro, trifluorométhyle, alcoxycarbonyle, alcoxycarbonylalkyle, alcoxycarbonylalcoxy, hydroxycarbonylalcoxy, carboxy, carboxyalkyle, dialkylaminoalkyle, hydroxy, amino, acétylamino, propionylamino, aminocarbonyle, alkylaminocarbonyle, dialkylaminocarbonyle, [N-alkyl-N-(dialkylaminoalkyl)amino]carbonyle, [(hydroxycarbonylalkyl)amino]carbonyle, [(alcoxycarbonylalkyl)amino]carbonyle, aminocarbonylamino, aminocarbonylaminoalkyle, alkylaminocarbonylamino, alcanoyle ou trifluorométhoxy,
par des groupes alkylèneimino à 5 à 7 chaînons, où un groupe méthylène en position 3, 4 ou 5 peut être remplacé par un atome d'oxygène ou un groupe méthylimino,
par des groupes alcoxy qui peuvent être substitués en position $\omega$ par un hétéroalicycle à 5 à 7 chaînons, où l'hétéroalicycle est lié par un atome de carbone ou d'azote et contient un ou deux hétéroatomes non liés directement l'un à l'autre choisis parmi l'oxygène et l'azote,
où une substitution multiple par des groupements cycliques ou par des groupements qui contiennent un carbo- ou hétérocycle est exclue et où les substituants peuvent être identiques ou différents,
et $R^1$ représente un groupe phényle, 1-naphtyle ou 2-naphtyle,
où les groupements aromatiques cités précédemment peuvent être mono-, di- ou trisubstitués par des atomes de fluor, de chlore ou de brome, par des groupes alkyle ramifiés ou non ramifiés, par des groupes alcoxy, trifluorométhyle, nitro, hydroxy, amino ou acétylamino et les substituants peuvent être identiques ou différents,
et où tous les groupes alkyle et alcoxy cités précédemment ainsi que les parties alkyle ou alkylène présentes dans les autres groupements cités peuvent comprendre, sauf indication contraire, 1 à 4 atomes de carbone,
leurs tautomères, leurs diastéréoisomères, leurs énantiomères et leurs sels.

**3.** Composés de formule générale I selon la revendication 1 où
R représente un hétérocycle diaza ou triaza mono-insaturé à 5 à 7 chaînons,
où les hétérocycles cités précédemment sont liés par un atome d'azote,
contiennent groupe carbonyle voisin d'un atome d'azote et
peuvent être substitués sur un atome de carbone par un groupe phényle ou
une double liaison oléfinique de l'un des hétérocycles insaturés cités précédemment peut être condensée avec un cycle benzène, pyridine ou quinoléine, et où les groupes phényle contenus dans R ainsi que les hétérocycles benzo- et pyridocondensés peuvent être mono-, di- ou trisubstitués en outre dans le squelette carboné par des atomes de fluor, de chlore ou de brome, par des groupes alkyle, dialkylaminoalcoxy, nitro, trifluorométhyle, alcoxycarbonyle, alcoxycarbonylalcoxy, hydroxycarbonylalcoxy, carboxy, hydroxy, aminocarbonyle, alkylaminocarbonyle, dialkylaminocarbonyle, [N-alkyl-N-(dialkylaminoalkyl)amino]carbonyle, [(hydroxycarbonylalkyl)amino]carbonyle, [(alcoxycarbonylalkyl)amino]carbonyle, alcanoyle ou trifluorométhoxy,
par des groupes alkylèneimino à 5 à 7 chaînons, où un groupe méthylène en position 3 ou 4 peut être remplacé par un atome d'oxygène ou un groupe méthylimino, par exemple 1-pyrrolidinyle, 1-pipéridinyle, 4-méthyl-1-pipérazinyle, 4-méthyl-1,4-diazacyclohept-1-yle ou 4-morpholinyle,
par des groupes alcoxy qui peuvent être substitués en position $\omega$ par un hétéroalicycle à 5 ou 6 chaînons, où

l'hétéroalicycle est lié par un atome de carbone et contient un atome d'oxygène en position 2 et un atome d'oxygène en position 2' ou est lié par un atome de carbone ou d'azote et contient un ou deux atomes d'azote non liés directement l'un à l'autre ou un atome d'oxygène et un atome d'azote qui sont séparés l'un de l'autre par au moins un groupe méthylène, par exemple méthoxy, éthoxy, propoxy, 2,5-dioxacyclopentylméthoxy, 2,6-dioxacyclohexylméthoxy, 2-(1-pyrrolidinyl)éthoxy, 2-(1-pipéridinyl)éthoxy, 2-(4-méthyl-1-pipérazinyl)éthoxy ou 2-(4-morpholinyl) éthoxy,
où une substitution multiple par des groupements cycliques ou des groupements qui contiennent un carbo- ou hétérocycle est exclue et où les substituants peuvent être identiques ou différents,
et $R^1$ représente un groupe phényle qui peut être mono-, di- ou trisubstitué par des atomes de fluor, de chlore ou de brome, par des groupes alcoxy, trifluorométhyle, nitro, hydroxy ou amino, où les substituants peuvent être identiques ou différents, où tous les groupes alkyle et alcoxy cités précédemment ainsi que les parties alkyle ou alkylène présentes dans les autres groupements cités peuvent comprendre, sauf indication contraire, 1 à 3 atomes de carbone,
leurs tautomères, leurs diastéréoisomères, leurs énantiomères et leurs sels.

**4.** Composés de formule générale I selon la revendication 1 où
R représente un groupe 3,4-dihydro-2(1H)-oxoquinazolin-3-yle, 1,3-dihydro-4-phényl-2H-2-oxoimidazol-1-yle, 2,4-dihydro-5-phényl-3(3H)-oxo-1,2,4-triazol-2-yle, 3,4-dihydro-2(1H)-oxopyrido[4,3-d]-pyrimidin-3-yle, 3,4-dihydro-2(1H)-oxopyrido[3,4-d]pyrimidin-3-yle ou 1,3-dihydro-2(2H)-oxoimidazo[4,5-c]quinolin-3-yle,
où les hétérocycles mono- et bicycliques cités précédemment peuvent être mono- ou disubstitués dans le squelette carboné par des atomes de fluor, de chlore ou de brome ou monosubstitués par un groupe 4-méthyl-1-pipérazinyle, 2,5-dioxacyclopentylméthoxy, méthoxy, 2-(4-morpholinyl)éthoxy, 2-diméthylaminoéthoxy, 3-diméthylaminopropoxy, méthoxycarbonylméthoxy, hydroxycarbonylméthoxy, nitro, trifluorométhyle, méthoxycarbonyle, carboxy, hydroxy, aminocarbonyle, diéthylaminocarbonyle, [N-(2-diméthylaminoéthyl)-N-méthylamino]carbonyle, [(méthoxycarbonylméthyl)amino]carbonyle ou [(hydroxycarbonylméthyl)amino]carbonyle,
et $R^1$ représente un groupe phényle qui peut être mono-, di- ou trisubstitué par des atomes de fluor, de chlore ou de brome, par des groupes hydroxy ou amino, où les substituants peuvent être identiques ou différents, par exemple le groupe 4-chlorophényle, 4-amino-3,5-dibromophényle ou 3,5-dibromo-4-hydroxyphényle, leurs tautomères, leurs diastéréoisomères, leurs énantiomères et leurs sels.

**5.** Composés de formule générale I selon la revendication 1 suivants :

(1) cis-3-{1-[2-(4-chlorobenzoyl)-cyclopropanecarbonyl]-4-pipéridinyl}-3,4-dihydro-2(1H)-quinazolinone

(2) trans-1-{1-[2-(4-amino-3,5-dibromobenzoyl)-cyclopropanecarbonyl]-4-pipéridinyl}-1,3-dihydro-4-(3-méthoxyphényl)-2(2H)-imidazolone

(3) trans-3-{1-[2-(4-amino-3,5-dibromobenzoyl)-cyclopropanecarbonyl]-4-pipéridinyl}-3,4-dihydro-2(1H)-quinazolinone

(4) trans-3-{1-[2-(4-amino-3,5-dibromobenzoyl)-cyclopropanecarbonyl]-4-pipéridinyl}-6-bromo-3,4-dihydro-2(1H)-quinazolinone

(5) trans-1-{1-[2-(4-amino-3,5-dibromobenzoyl)-cyclopropanecarbonyl]-4-pipéridinyl}-1,3-dihydro-4-phényl-2(2H)-imidazolone

(6)trans-1-{1-[2-(4-amino-3,5-dibromobenzoyl)-cyclopropanecarbonyl]-4-pipéridinyl}-1,3-dihydro-4-[3-(trifluorométhyl)phényl]-2(2H)-imidazolone

(7) trans-1-{1-[2-(4-amino-3,5-dibromobenzoyl)-cyclopropanecarbonyl]-4-pipéridinyl}-1,3-dihydro-4-(3-hydroxyphényl)-2(2H)-imidazolone

(8) trans-3-{1-[2-(4-amino-3,5-dibromobenzoyl)-cyclopropanecarbonyl]-4-pipéridinyl}-3,4-dihydro-6-hydroxy-2(1H)-quinazolinone

(9) trans-3-{1-[2-(4-amino-3,5-dibromobenzoyl)-cyclopropanecarbonyl]-4-pipéridinyl}-3,4-dihydro-6-[(1,3dioxolan-2-yl)méthoxy]-2(1H)-quinazolinone

(10) trans-1-{1-[2-(4-amino-3,5-dibromobenzoyl)-cyclopropanecarbonyl]-4-pipéridinyl}-4-(3,4-dichlorophényl)-1,3-dihydro-2(2H)-imidazolone

(11) trans-3-{1-[2-(4-amino-3,5-dibromobenzoyl)-cyclopropanecarbonyl]-4-pipéridinyl}-3,4-dihydro-2(1H)-pyrido[4,3-d]pyrimidinone

(12) trans-1-{1-[2-(4-amino-3,5-dibromobenzoyl)-cyclopropanecarbonyl]-4-pipéridinyl}-1,3-dihydro-4-(2-méthoxyphényl)-2(2H)-imidazolone

(13) trans-1-{1-[2-(4-amino-3,5-dibromobenzoyl)-cyclopropanecarbonyl]-4-pipéridinyl}-4-(3-chlorophényl)-1,3-dihydro-2(2H)-imidazolone

(14) trans-1-{1-[2-(4-amino-3,5-dibromobenzoyl)-cyclopropanecarbonyl]-4-pipéridinyl}-1,3-dihydro-4-(3-nitrophényl)-2(2H)-imidazolone

(15) trans-3-{1-[2-(4-amino-3,5-dibromobenzoyl)-cyclopropanecarbonyl]-4-pipéridinyl}-3,4-dihydro-2(1H)-pyrido[3,4-d]pyrimidinone

(16) trans-3-{1-[2-(4-amino-3,5-dibromobenzoyl)-cyclopropanecarbonyl]-4-pipéridinyl}-3,4-dihydro-6-[2-(diméthylamino)éthoxy]-2(1H)-quinazolinone

(17) trans-3-{1-[2-(4-amino-3,5-dibromobenzoyl)-cyclopropanecarbonyl]-4-pipéridinyl}-3,4-dihydro-6-(4-méthyl-1-piperazinyl)-2(1H)-quinazolinone

(18) trans-1-{1-[2-(4-amino-3,5-dibromobenzoyl)-cyclopropanecarbonyl]-4-pipéridinyl}-1,3-dihydro-4-[2-(trifluorométhyl)phényl]-2(2H)-imidazolone

(19) trans-3-{1-[2-(4-amino-3,5-dibromobenzoyl)-cyclopropanecarbonyl]-4-pipéridinyl}-3,4-dihydro-6-(3-(diméthylamino)propoxy]-2(1H)-quinazolinone

(20) trans-3-{1-[2-(3,5-dibromo-4-hydroxybenzoyl)-cyclopropanecarbonyl]-4-pipéridinyl}-3.,4-dihydro-2(1H)-quinazolinone

(21) trans-1-{1-[2-(3,5-dibromo-4-hydroxybenzoyl)-cyclopropanecarbonyl]-4-pipéridinyl}-1,3-dihydro-4-phényl-2(2H)-irnidazolone

(22) trans-3-{1-[2-(4-amino-3,5-dibromobenzoyl)-cyclopropanecarbonyl]-4-pipéridinyl}-3,4-dihydro-6-(méthoxycarbonylméthoxy)-2(1H)-quinazolinone

(23) trans-3-{1-[2-(4-amino-3,5-dibromobenzoyl)-cyclopropanecarbonyl]-4-pipéridinyl}-3,4-dihydro-6-(hydroxycarbonylméthoxy)-2(1H)-quinazolinone

(24) trans-3-{1-[2-(4-amino-3,5-dibromobenzoyl)-cyclopropanecarbonyl]-4-pipéridinyl}-3,4-dihydro-6-[2-(4-morpholinyl)éthoxy]-2(1H)-quinazolinone

(25) trans-3-{1-[2-(4-amino-3,5-dibromobenzoyl)-cyclopropanecarbonyl]-4-pipéridinyl}-3,4-dihydro-7-méthoxy-2(1H)-quinazolinone

(26) trans-3-{1-[2-(4-amino-3,5-dibromobenzoyl)-cyclopropanecarbonyl]-4-pipéridinyl}-3,4-dihydro-7-(méthoxycarbonylméthoxy)-2(1H)-quinazolinone

(27) trans-3-{1-[2-(4-amino-3,5-dibromobenzoyl)-cyclopropanecarbonyl]-4-pipéridinyl}-7-carboxy-3,4-dihydro-2(1H)-quinazolinone

(28) trans-3-{1-[2-(4-amino-3,5-dibromobenzoyl)-cyclopropanecarbonyl]-4-pipéridinyl}-7-méthoxycarbonyl-3,4-dihydro-2(1H)-quinazolinone

(29) trans-3-{1-[2-(4-amino-3,5-dibromobenzoyl)-cyclopropanecarbonyl]-4-pipéridinyl}-1,3-dihydro-2(2H)-imidazo[4,5-c]quinolinone

(30) trans-3-{1-[2-(4-amino-3,5-dibromobenzoyl)-cyclopropanecarbonyl]-4-pipéridinyl}-3,4-dihydro-7-{[(méthoxycarbonylméthyl)amino]carbonyl}-2(1H)-quinazolinone

(31) trans-3-{1-[2-(4-amino-3,5-dibromobenzoyl)-cyclopropanecarbonyl]-4-pipéridinyl}-3,4-dihydro-7-{[N-(2-diméthylaminoéthyl)-N-méthylamino]-carbonyl}-2(1H)-quinazolinone

(32) trans-3-{1-[2-(4-amino-3,5-dibromobenzoyl)-cyclopropanecarbonyl]-4-pipéridinyl}-7-diéthylaminocarbonyl-3,4-dihydro-2(1H)-quinazolinone

(33) trans-7-aminocarbonyl-3-{1-[2-(4-amino-3,5-dibromobenzoyl)-cyclopropanecarbonyl]-4-pipéridinyl}-3,4-dihydro-2(1H)-quinazolinone

(34) trans-3-{1-[2-{4-amino-3,5-dibromobenzoyl)-cyclopropanecarbonyl]-4-pipéridinyl}-3,4-dihydro-7-{[(hydroxycarbonylméthyl)amino]carbonyl}-2(1H)-quinazolinone.

(35) trans-1-{1-[2-(4-amino-3,5-dibromobenzoyl)-cyclopropanecarbonyl]-4-pipéridinyl}-2,4-dihydro-5-phényl-3(3H)-1,2,4-triazolone,

ainsi que leurs sels.

**6.** Composés de formule générale I selon la revendication 1 suivants :

(a) trans-1-{1-[2-(4-amino-3,5-dibromobenzoyl)-cyclopropanecarbonyl]-4-pipéridinyl}-1,3-dihydro-4-(3-méthoxyphényl)-2(2H)-imidazolone,

(b) trans-3-{1-[2-(4-amino-3,5-dibromobenzoyl)-cyclopropanecarbonyl]-4-pipéridinyl}-3,4-dihydro-2(1H)-quinazolinone,

(c) trans-1-{1-[2-(4-amino-3,5-dibromobenzoyl)-cyclopropanecarbonyl]-4-pipéridinyl}-1,3-dihydro-4-phényl-2 (2H)-imidazolone,

(d) trans-1-{1-[2-(4-amino-3,5-dibromobenzoyl)-cyclopropanecarbonyl]-4-pipéridinyl}-1,3-dihydro-4-(3-hydroxyphényl)-2(2H)-imidazolone,

(e) trans-3-{1-[2-(4-amino-3,5-dibromobenzoyl)-cyclopropanecarbonyl]-4-pipéridinyl}-3,4-dihydro-6-hydroxy-2(1H)-quinazolinone,

(f) trans-3-{1-[2-(4-amino-3,5-dibromobenzoyl)-cyclopropanecarbonyl]-4-pipéridinyl}-3,4-dihydro-6-[(1,3-dioxolan-2-yl)méthoxy]-2(1H)-quinazolinone,

(g) trans-1-{1-[2-(4-amino-3,5-dibromobenzoyl)-cyclopropanecarbonyl]-4-pipéridinyl}-4-(3-chlorophényl)-1,3-dihydro-2(2H)-imidazolone,

(h) trans-3-{1-[2-(4-amino-3,5-dibromobenzoyl)-cyclopropanecarbonyl]-4-pipéridinyl}-3,4-dihydro-6-[3-(diméthylamino)propoxy]-2(1H)-quinazolinone,

(i) trans-3-{1-[2-(4-amino-3,5-dibromobenzoyl)-cyclopropanecarbonyl]-4-pipéridinyl}-3,4-dihydro-6-(méthoxycarbonylméthoxy)-2(1H)-quinazolinone,

(j) trans-3-{1-[2-(4-amino-3,5-dibromobenzoyl)-cyclopropanecarbonyl]-4-pipéridinyl}-3,4-dihydro-6-(hydroxycarbonylméthoxy)-2(1H)-quinazolinone et

(k) trans-1-{1-[2-(4-amino-3,5-dibromobenzoyl)-cyclopropanecarbonyl]-4-pipéridinyl}-2,4-dihydro-5-phényl-3 (3H)-1,2,4-triazolone

ainsi que leurs sels.

7. Sels physiologiquement acceptables des composés selon au moins l'une des revendications 1 à 6 avec des acides ou des bases inorganiques ou organiques.

8. Médicament contenant un composé selon au moins l'une des revendications 1 à 6 ou un sel physiologiquement acceptable selon la revendication 7 outre éventuellement un ou plusieurs supports et/ou diluants inertes.

9. Utilisation d'un composé selon au moins l'une des revendications 1 à 7 pour la production d'un médicament qui présente des propriétés antagonistes de CGRP.

10. Utilisation d'un composé selon au moins l'une des revendications 1 à 7 pour la production d'un médicament qui convient pour le traitement aigu et prophylactique des céphalées, pour le traitement du diabète sucré non-insulinodépendant, des maladies cardiovasculaires, des maladies de la peau, des maladies inflammatoires, de la rhinite allergique, de l'asthme, des maladies qui se développent avec un élargissement excessif des vaisseaux et une irrigation sanguine réduite qui en résulte, de la tolérance à la morphine ou pour la lutte contre les bouffées de chaleur ménopausiques.

11. Procédé de production d'un médicament selon la revendication 8 **caractérisé en ce que**, par voie non chimique, un composé selon au moins l'une des revendications 1 à 7 est incorporé dans un ou plusieurs supports et/ou diluants inertes.

12. Procédé de production des composés de formule générale I selon les revendications 1 à 7 **caractérisé en ce que**

a) un acide carboxylique de formule générale

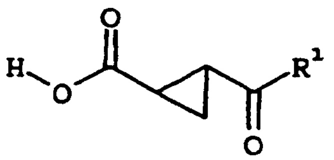

(II),

où
$R^1$ est défini comme indiqué dans les revendications 1 à 6,
est couplé avec un composé de formule générale

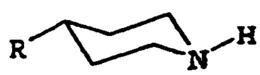

(III),

où
R est défini comme indiqué dans les revendications 1 à 6, ou
b) un composé de formule générale

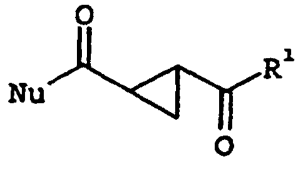

(IV),

où
$R^1$ est défini comme indiqué dans les revendications 1 à 6 et Nu représente un groupe partant,
est couplé avec un composé de formule générale

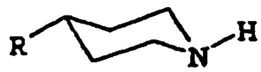

(III),

où
R est défini comme dans les revendications 1 à 6, ou
c) un composé de formule générale

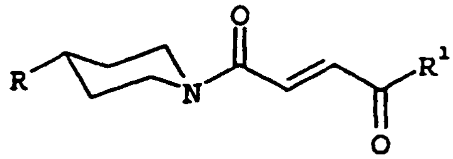

(V),

où
R et $R^1$ sont définis comme indiqué dans les revendications 1 à 6 est cyclopropanylé ou
d) pour la production d'un composé de formule générale I où au moins l'un des groupements R et $R^1$ contient un ou plusieurs groupes carboxy,
un ester d'acide carboxylique de formule générale

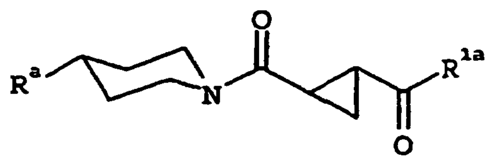

(Ia),

où
$R^a$ et $R^{1a}$ possèdent les significations indiquées dans les revendications 1 à 6 pour R et $R^1$ avec la condition qu'au moins l'un de ces groupements contienne un ou plusieurs groupes alcoxycarbonyle, est soumis à une saponification alcaline
puis, si on le souhaite, un sel ainsi obtenu est converti par traitement avec un acide organique ou inorganique dilué en l'acide de base ou
e) pour la production d'un composé de formule générale I où
le groupement R est mono-, di- ou trisubstitué de la même façon dans le squelette carboné par un groupe aminocarbonyle, alkylaminocarbonyle, dialkylaminocarbonyle, [N-alkyl-N-(dialkylaminoalkyl)amino]carbonyle, hydroxycarbonylalkylaminocarbonyle, alcoxycarbonylalkylaminocarbonyle, (4-morpholinyl)carbonyle, (1-pyrrolidinyl)carbonyle, (1-pipéridinyl)carbonyle, (hexahydro-1-azépinyl)carbonyle ou (4-méthyl-1-pipérazinyl) carbonyle, un composé de formule générale

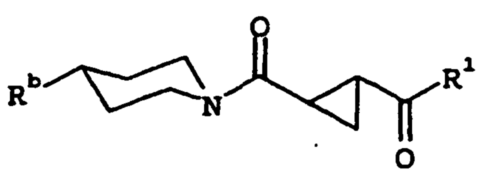

(Ib),

où
$R^1$ est défini comme indiqué dans les revendications 1 à 6 et le groupement $R^b$ a les significations indiquées dans les revendications 1 à 6 pour R avec la condition qu'il soit mono-, di- ou trisubstitué dans le squelette carboné par le groupe carboxy,
est couplé avec l'ammoniac, avec une alkylamine, N-alkyl-N-(dialkylaminoalkyl)amine, hydroxycarbonylalkylamine, alcoxycarbonylalkylamine ou dialkylamine correspondante puis
si on le souhaite un composé de formule générale I ainsi obtenu est résolu en ses diastéréoisomères et/ou énantiomères et/ou
un composé de formule générale I ainsi obtenu est converti en ses sels, en particulier en ses sels physiologiquement acceptables.